# EUROPÄISCHE PATENTSCHRIFT

(11) **EP 1 173 519 B1**
(45) Veröffentlichungstag und Bekanntmachung des Hinweises auf die Patenterteilung: **20.08.2003**
(21) Anmeldenummer: 00922654.9
(22) Anmeldetag: 19.04.2000
(51) Int. Cl.: C09B 11/00, G01N 33/533, G01N 33/58, C07H 21/00, C09B 11/02

(54) **NEUE CARBOPYRONIN-FLUORESZENZ-FARBSTOFFE**
NOVEL CARBOPYRONINE FLUORESCENCE DYES
NOUVEAUX COLORANTS FLUORESCENTS DE CARBOPYRONINE

(30) Priorität: 27.04.1999 DE 19919119
(43) Veröffentlichungstag der Anmeldung: 23.01.2002
(73) Patentinhaber: DREXHAGE, Karl-Heinz, D-57076 Siegen (DE)
(72) Erfinder: DREXHAGE, Karl-Heinz, D-57076 Siegen (DE); ARDEN-JACOB, Jutta, D-90513 Zirndorf (DE); FRANTZESKOS, Jörg, D-57482 Wenden (DE); ZILLES, Alexander, West Yorkshire, Leeds LS7 3ED (GB)
(74) Vertreter: Weiss, Wolfgang, Dipl.-Chem. Dr.
(86) Internationale Anmeldenummer: EP0003568
(87) Internationale Veröffentlichungsnummer: WO00064986

(56) Entgegenhaltungen:
- EP-A- 0 543 333
- US-A- 5 366 860
- C. AARON AND C.C. BARKER: "Steric Effects in Di- and Tri-arylmethane Dyes. Part X." JOURNAL OF THE CHEMICAL SOCIETY, SECTION B: PHYSICAL ORGANIC CHEMISTRY., Nr. 2, 1971, Seiten 319-324, XP002140950 CHEMICAL SOCIETY. LETCHWORTH., GB
- G. HALLAS: "Electronic Absorption Spectrum of the alpha-1-Adamantyl Derivative of Michler's Hydrol Blue" JOURNAL OF THE CHEMICAL SOCIETY, SECTION B: PHYSICAL ORGANIC CHEMISTRY., Nr. 1, 1967, Seiten 91-92, XP002140951 CHEMICAL SOCIETY. LETCHWORTH., GB
- R.W. CASTELINO AND G. HALLAS: "Electronic Absorption Spectra of Some Analogues and Derivatives of Michler's Ketone" JOURNAL OF THE CHEMICAL SOCIETY, SECTION B: PHYSICAL ORGANIC CHEMISTRY., Nr. 7, 1971, Seiten 1468-1471, XP002140952 CHEMICAL SOCIETY. LETCHWORTH., GB

## Beschreibung

Die Erfindung betrifft die Verwendung von Carbopyroninverbindungen der allgemeinen Formel (I) als Markierungsgruppen in Verfahren zum Nachweis von Analyten, neue Carbopyroninverbindungen sowie ein Verfahren zur Herstellung dieser Verbindungen.

In der chemischen, medizinischen und biologischen Analytik werden Farbstoffe als Markierungs- bzw. Nachweisgruppen verwendet. Insbesondere Fluoreszenzfarbstoffe haben in den letzten Jahren an Bedeutung gewonnen und andere vielfach kostenintensive Verfahren verdrängt, die beispielsweise Radioisotope zur Markierung verwenden.

Insbesondere im Bereich der DNA-Sequenzierung haben sich fluorometrische Verfahren in den letzten Jahren durchgesetzt und die bis dahin üblichen Verfahren, die radioaktive Isotope verwenden, fast völlig verdrängt.

Trotz der Verfügbarkeit von verschiedenen Fluoreszenzfarbstoffen, wie beispielsweise FITC (Fluoresceinisothiocyanat), FLUOS (Fluorescein-N-hydroxysuccinimidester), Rhodamin-Derivate etc., konnten bisher die Probleme durch Hintergrundfluoreszenz, unspezifische Bindungsphänomene sowie Notwendigkeit kostenintensiver Meßapparaturen nicht in befriedigender Weise gelöst werden.

Durch Hintergrundfluoreszenz und unspezifische Bindungen wird die Empfindlichkeit und Genauigkeit der Messungen verringert. Des weiteren liegt bei verfügbaren Fluoreszenzfarbstoffen das Absorptionsmaximum in Bereichen, die die Verwendung kostengünstiger und klein dimensionierbarer Lichtquellen, wie beispielsweise He-Ne-Laser und Laserdioden, nicht ermöglichen.

Eine Aufgabe der vorliegenden Erfindung bestand somit darin, Fluoreszenzfarbstoffe bereitzustellen, die als Markierungsgruppe in Nachweisverfahren von Analyten eingesetzt werden können und die Nachteile des Standes der Technik zumindest teilweise vermeiden.

Diese Aufgabe wurde gelöst durch die Verwendung von Verbindungen der allgemeinen Formel (I) 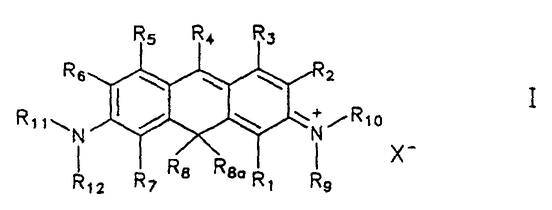 als Markierungsgruppen in einem Verfahren zum Nachweis eines Analyten, wobei
R₁, R₂, R₃, R₄, R₅, R₆ und R₇ jeweils unabhängig Wasserstoff, Halogen, eine Hydroxy-, Amino-, Sulfo- oder Carboxy- oder Aldehydgruppe oder eine gesättigte oder ungesättigte, geradkettige, verzweigte oder cyclische Kohlenwasserstoffgruppe mit bis zu 20 C-Atomen bedeuten, wobei die Kohlenwasserstoffgruppen Alkyl-, Alkenyl-, Alkinyl-, Cycloalkyl-, Aryl-, insbesondere Phenyl-, oder/und Heteroarylreste umfassen und gegebenenfalls Heteroatome wie Sauerstoff-, Schwefel- oder Stickstoffatome oder/und mehrere Substituenten, vorzugsweise ausgewählt aus Halogenen, Hydroxy-, Amino-, Sulfo-, Phospho-, Carboxy-, Aldehyd-, C₁-C₄-Alkoxy- oder/und C₁-C₄-Alkoxycarbonylgruppen enthalten, oder einer oder mehrere der Reste R₁-R₇ jeweils mit benachbarten Substituenten ein Ringsystem bilden, das eine oder mehrere Mehrfachbindungen enthalten kann,
R₈ und R₈ₐ jeweils unabhängig eine gesättigte oder ungesättigte, geradkettige, verzweigte oder cyclische Kohlenwasserstoffgruppe mit bis zu 20 Kohlenstoffatomen, z.B. eine C₁-C₆-Alkylgruppe, insbesondere Methyl, Ethyl, Propyl oder/und Butyl, oder eine Aryl- oder Heteroarylgruppe, insbesondere Phenyl, bedeuten, die gegebenenfalls Heteroatome wie Sauerstoff-, Schwefel- oder Stickstoffatome oder/und einen oder mehrere Substituenten, vorvorzugsweise ausgewählt aus Halogenen, Hydroxy-, Amino-, Sulfo-, Phospho-, Carboxy-, Aldehyd-, C₁-C₄-Alkoxy- oder/und C₁-C₄-Alkoxycarbonylgruppen, enthalten, oder auch R₈ und R₈ₐ ein Ringsystem bilden können, R_{9,} R₁₀, R₁₁ und R₁₂ jeweils unabhängig Wasserstoff oder eine gesättigte oder ungesättigte, geradkettige, verzweigte oder cyclische Kohlenwasserstoffgruppe mit bis zu 20 C-Atomen, z.B. Polyether, Phenyl, Phenylalkyl mit 1-3 C-Atomen in der Kette bedeuten, wobei die Kohlenwasserstoffgruppen gegebenenfalls Heteroatome wie Sauerstoff-, Schwefel- oder Stickstoffatome oder/und einen oder mehrere Substituenten, vorzugsweise ausgewählt aus Halogenen, Hydroxy-, Amino-, Sulfo-, Phospho-, Carboxy-, Carbonyl-, Alkoxy- oder/und Alkoxycarbonylgruppen enthalten können,
oder einer oder mehrere der Reste R₉-R₁₂ jeweils mit benachbarten Substituenten ein Ringsystem bilden, das eine oder mehrere Mehrfachbindungen enthalten kann,
wobei -N(R₁₁)(R₁₂) oder/und =N(R₉)(R₁₀) durch -OR⁹ oder/und =O ersetzt sein können,
und X gegebenenfalls zum Ladungsausgleich vorhandene Anionen bedeutet.

Die Verbindungen der allgemeinen Formel (I) können als Markierungsgruppen in Verfahren zur qualitativen oder/und quantitativen Bestimmung eines Analyten eingesetzt werden. Diese Bestimmung kann in wässrigen Flüssigkeiten, z.B. Proben von Körperflüssigkeiten wie etwa Blut, Serum, Plasma oder Urin, Abwasserproben oder Lebensmitteln, durchgeführt werden. Das Verfahren kann sowohl als Naßtest, z.B. in einer Küvette, oder als Trockentest auf einem entsprechenden Reagenzträger durchgeführt werden. Die Bestimmung des Analyten kann hierbei mittels einer einzigen Reaktion oder durch eine Sequenz von Reaktionen erfolgen. Überraschenderweise zeigte die Verwendung von Verbindungen der allgemeinen Formel (I) sehr gute Ergebnisse in chemischen und insbesondere in medizinischen und biologischen Nachweisverfahren zur Bestimmung eines Analyten, speziell in Nukleinsäure-Sequenzierverfahren sowie in der Proteinanalytik.

Die Verbindungen der allgemeinen Formel (I) können in allen dem Fachmann bekannten chemischen, medizinischen und biologischen Nachweisverfahren, in denen Fluoreszenzfarbstoffe als Markierungsgruppe geeignet sind, verwendet werden. Hierzu werden die Verbindungen der allgemeinen Formel (I) im allgemeinen kovalent an einen für den nachzuweisenden Analyten spezifischen Rezeptor gekoppelt. Dies geschieht mit allgemein bekannten Verfahren. Der spezifische Rezeptor kann jede geeignete Verbindung oder jedes geeignete Molekül sein, vorzugsweise ist es ein Peptid, ein Polypeptid oder eine Nukleinsäure. Die Verbindungen oder Konjugate dieser Verbindungen können beispielsweise in Nukleinsäure-Hybridisierungsverfahren, insbesondere für die Sequenzierung von Nukleinsäuren oder immunchemischen Verfahren verwendet werden. Derartige Verfahren sind beispielsweise beschrieben in Sambrook et al., Molecular Cloning, A Laboratory Manual, 1989, Cold Spring Harbor.

Eine weitere Aufgabe der vorliegenden Erfindung bestand darin, neue Carbopyronin-Verbindungen bereitzustellen, die insbesondere zur Verwendung als Markierungsgruppe in Nachweisverfahren von Analyten geeignet sind, mit einfachen und kostengünstigen Verfahren hergestellt werden können, problemlos handhabbar sind und die Nachteile des Standes der Technik zumindest teilweise vermeiden.

Diese Aufgabe wurde gelöst durch eine Verbindung der allgemeinen Formel (I) 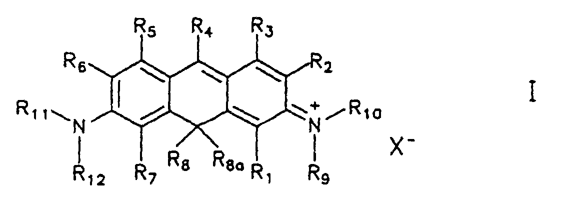 wobei R₁-R₁₂ und X die oben angegebenen Bedeutungen besitzen, mit der Maßgabe, daß, wenn R₁-R₃ und R₅-R₇ Wasserstoff sind und R₈, R₈ₐ und R₉-R₁₂ Methyl sind,
R₄ nicht Wasserstoff, Hydroxyl, Methyl, Isopropyl, t-Butyl, Phenyl, o-Tolyl, p-Tolyl, 2,6-Dimethylphenyl, 2-t-Butylphenyl, 2-Isopropenylphenyl oder 4-Dimethylaminophenyl ist.

Ein Vorteil der Verbindungen (I) ist, daß durch eine fast beliebige Substituentenvariation die Eigenschaften einzelner Verbindungen, z.B. die spektroskopischen Eigenschaften, die Lage der Absorptions- und Fluoreszenzmaxima, die Löslichkeitseigenschaften, die Fluoreszenz-Quantenausbeute und -Abklingzeit, stark variiert und somit wie gewünscht ausgewählt werden können. Auf diese Weise können Interferenzen mit Störsubstanzen in Proben wie Serum, Blut oder Plasma etc. vermindert oder sogar ganz vermieden werden. Die Herstellung einiger Verbindungen der Formel (I) kann nach an sich bekannten Verfahren erfolgen. Vorzugsweise erfolgt die Synthese jedoch nach einem neuen, im folgenden beschriebenen Verfahren, das besonders einfach und kostengünstig ist.

In einer bevorzugten Klasse der Verbindungen (I) sind R₆ mit R₁₁ oder/und R₇ mit R₁₂, R₁ mit R₁₀ oder/und R₂ mit R₉ verbrückt und bilden ein Ringsystem, das eine oder mehrere Mehrfachbindungen enthalten kann. Vorzugsweise enthält das Ringsystem einen oder mehrere 5- oder 6-gliedrige Ringe.

R₄ bedeutet vorzugsweise Wasserstoff, C₁-C₆-Alkyl oder einen ein aromatisches Ringsystem enthaltenden Rest, z.B. einen Carboxy- oder/und Halogengruppen enthaltenden Rest wie 2-Carboxy-phenyl, 2-Carboxytetrachlor-phenyl oder Pentafluorphenyl. R₈ und R₈ₐ sind vorzugsweise jeweils unabhängig Methyl, Ethyl oder/und gegebenenfalls substituiertes Phenyl.

Beispiele für besonders bevorzugte Verbindungsklassen sind in den allgemeinen Formeln IVa bis IVe dargestellt: 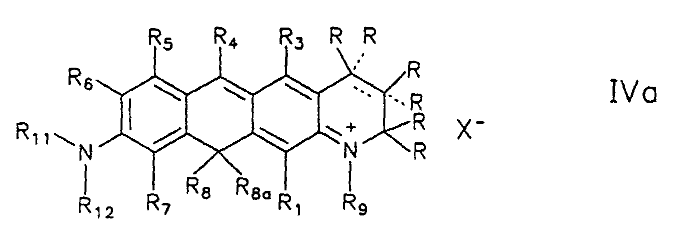 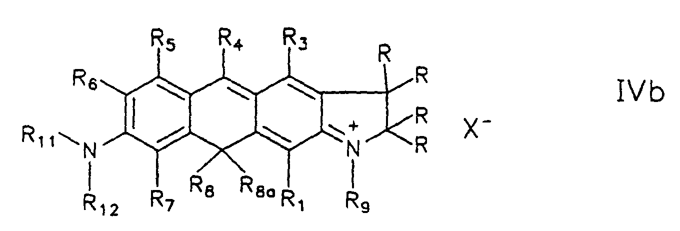 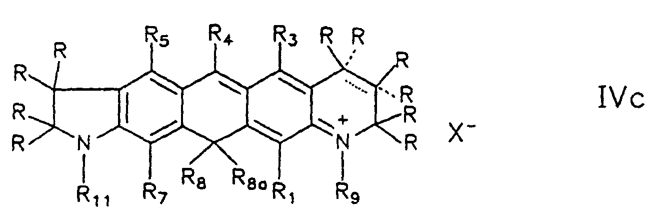 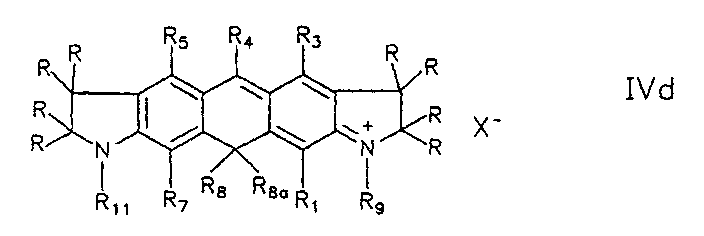 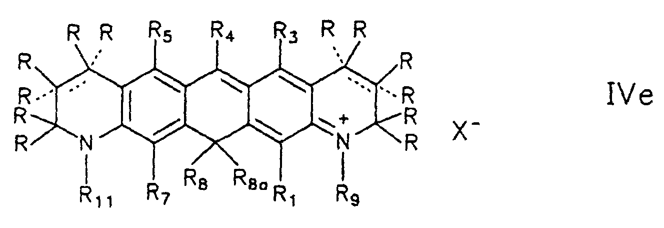 worin die gestrichelten Linien gegebenenfalls Doppelbindungen bedeuten, bei deren Vorhandensein die über eine gestrichelte Linie gebundenen Reste R fehlen,
R₁, R₃, R₄, R₅, R₆, R₇, R₈, R₈ₐ, R₉, R₁₁, R₁₂ und X wie oben definiert sind, und R bei jedem Auftreten gleich oder verschieden sein kann und wie R₁-R₇ oben definiert ist.

Die Verbindungen weisen vorzugsweise eine zur kovalenten Kopplung fähige Gruppe auf, z.B. -COOH, -NH₂, -OH oder/und -SH. Über diese Kopplungsgruppe kann die Verbindung nach bekannten Methoden an einen Träger oder/und an ein Biomolekül gekoppelt werden. Der Träger kann aus jedem geeigneten, insbesondere für Nachweisverfahren geeigneten Material bestehen, z.B. aus porösem Glas, Kunststoffen, lonenaustauschharzen, Dextranen, Cellulose, Cellulosederivaten oder/und hydrophilen Polymeren. Die Biomoleküle werden vorzugsweise ausgewählt aus Peptiden, Polypeptiden, Nukleotiden, Nukleosiden, Nukleinsäuren, Nukleinsäureanaloga oder/und Haptenen.

Überraschenderweise werden die Absorptionsmaxima und die Fluoreszenzquantenausbeute durch eine Kopplung der erfindungsgemäßen Verbindungen an die oben genannten Träger und Biomoleküle nicht wesentlich verändert.

Konkrete Beispiele für erfindungsgemäße Verbindungen sind in der folgenden Tabelle 1 dargestellt.

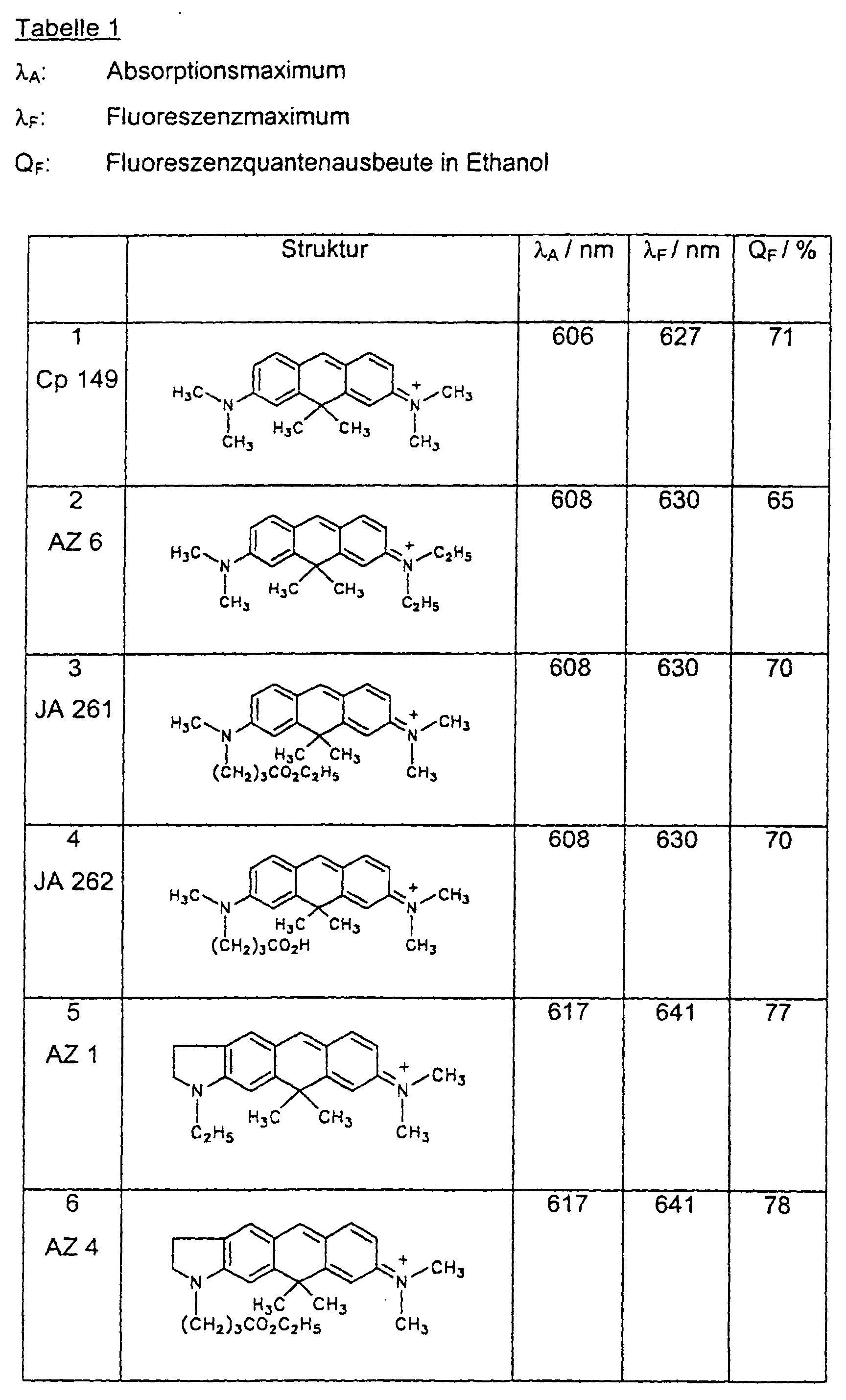

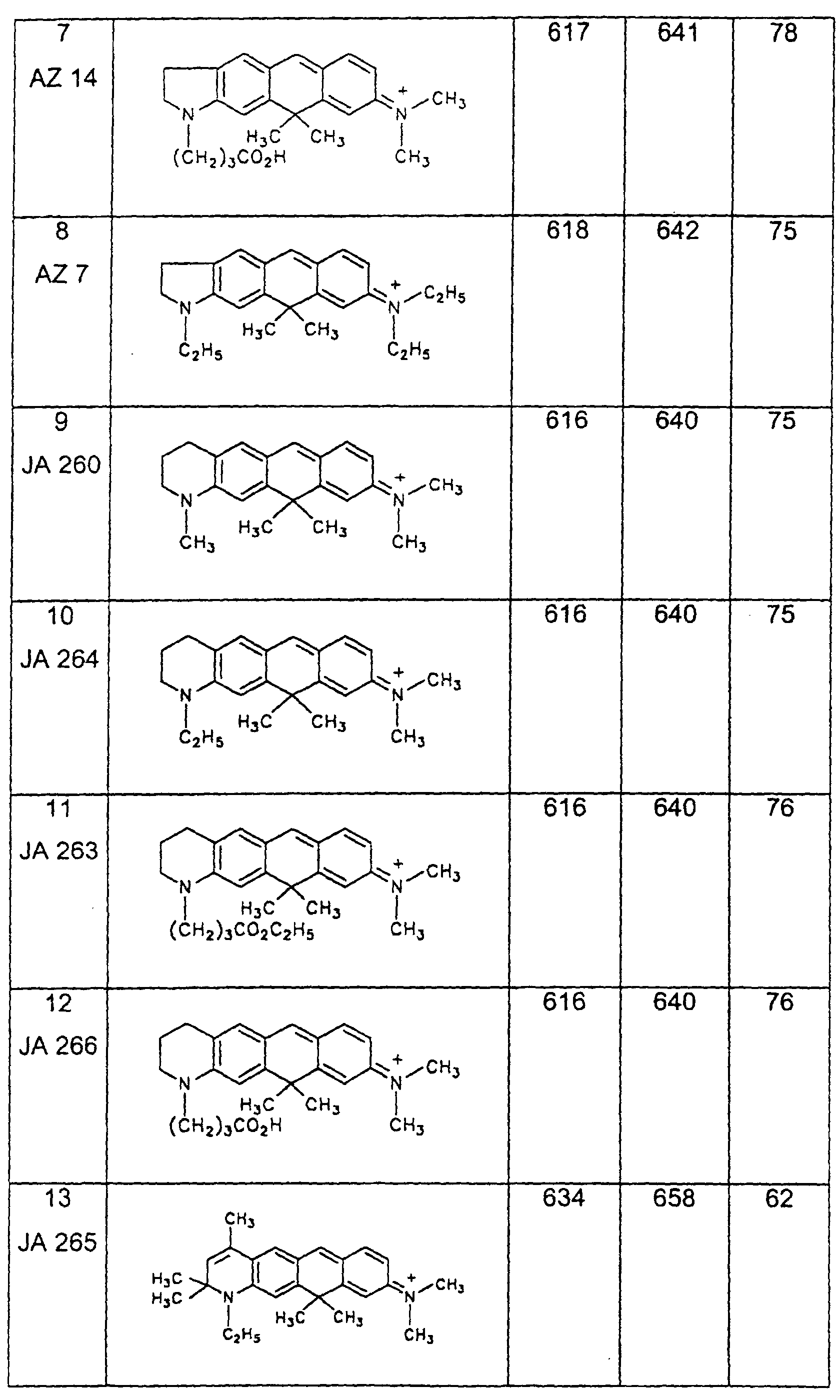

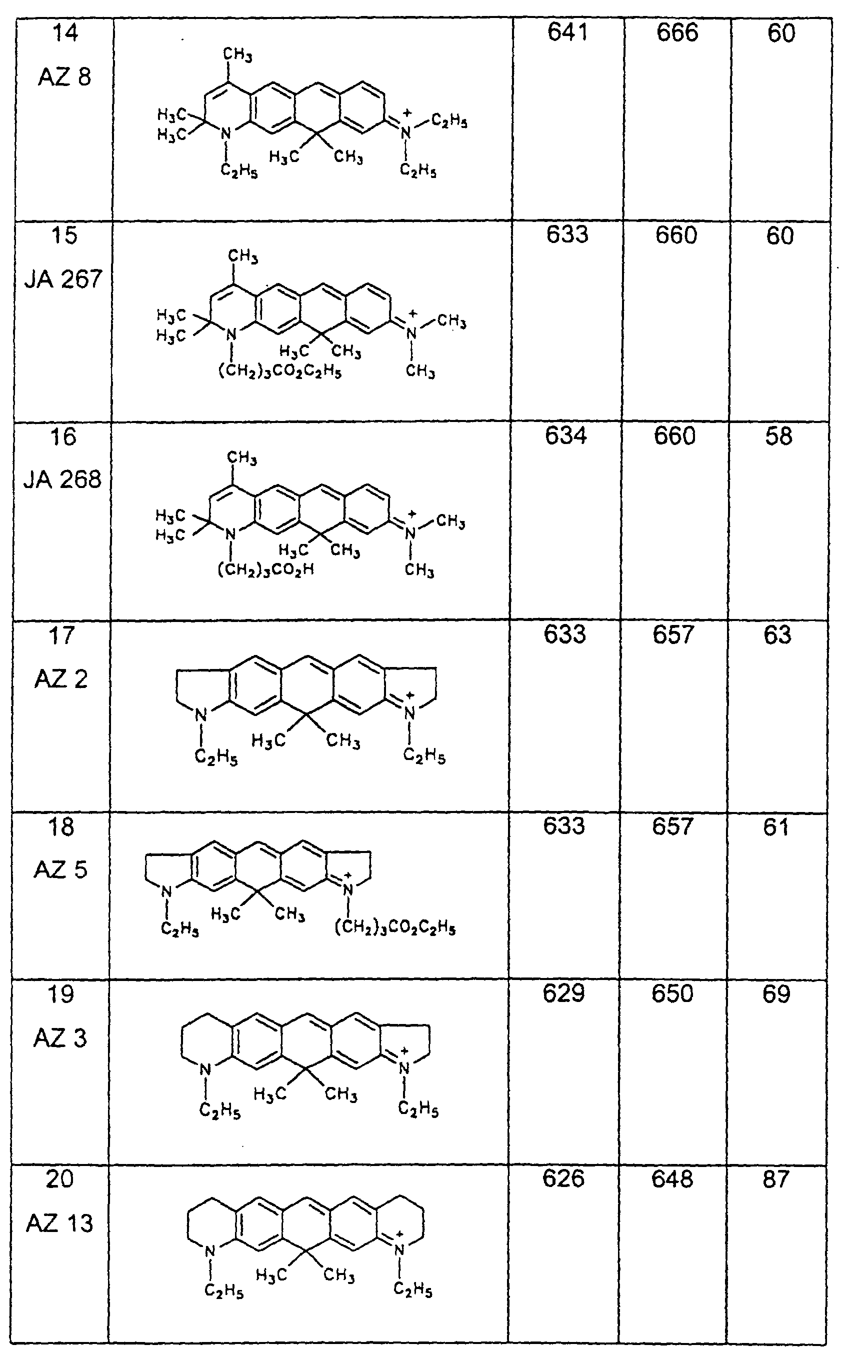

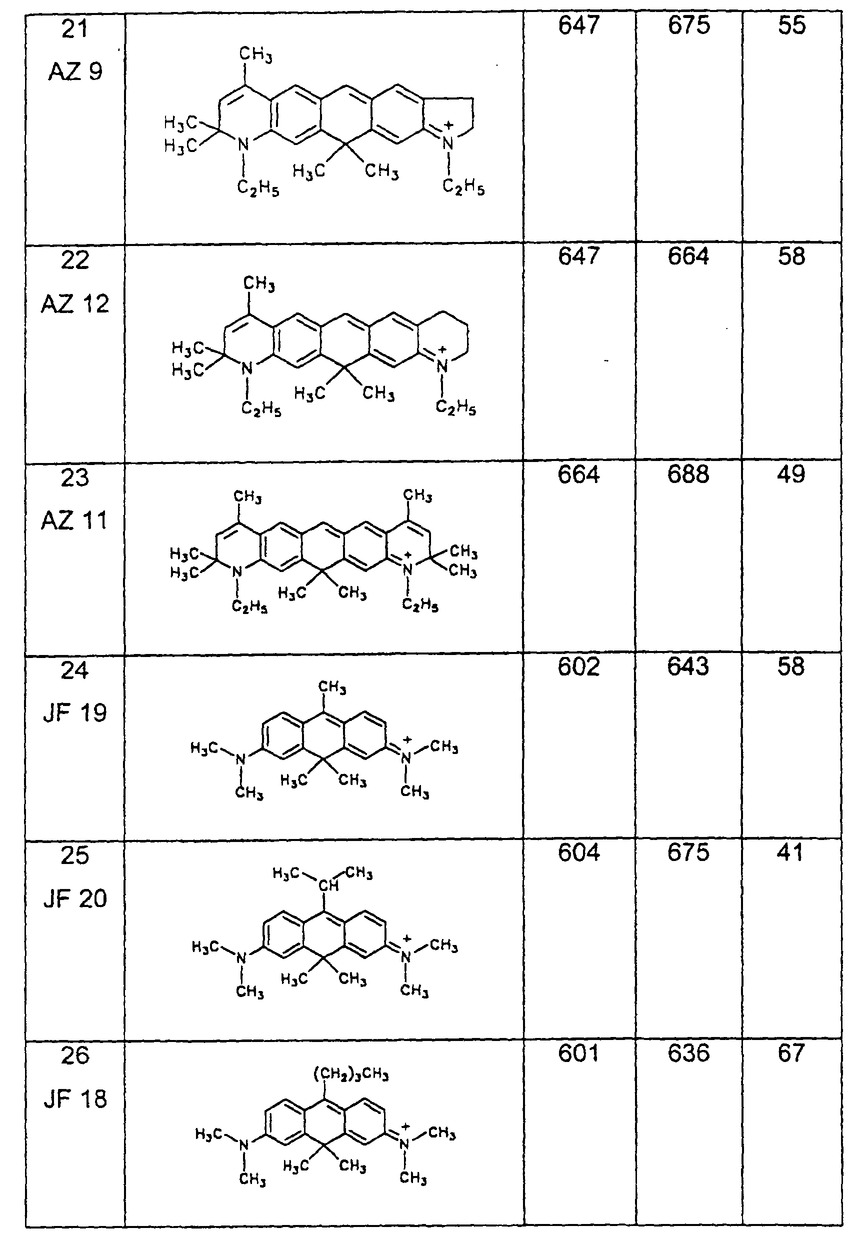

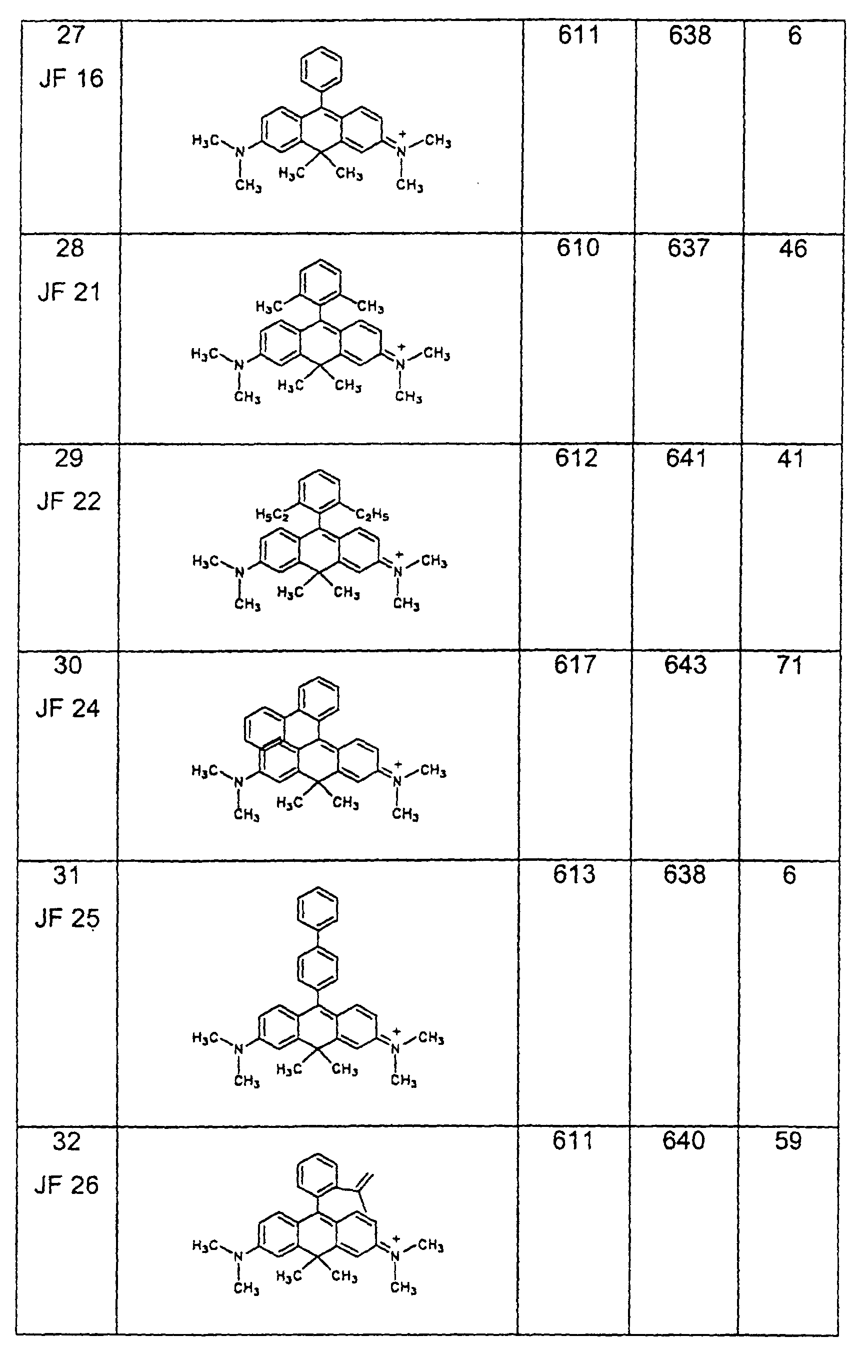

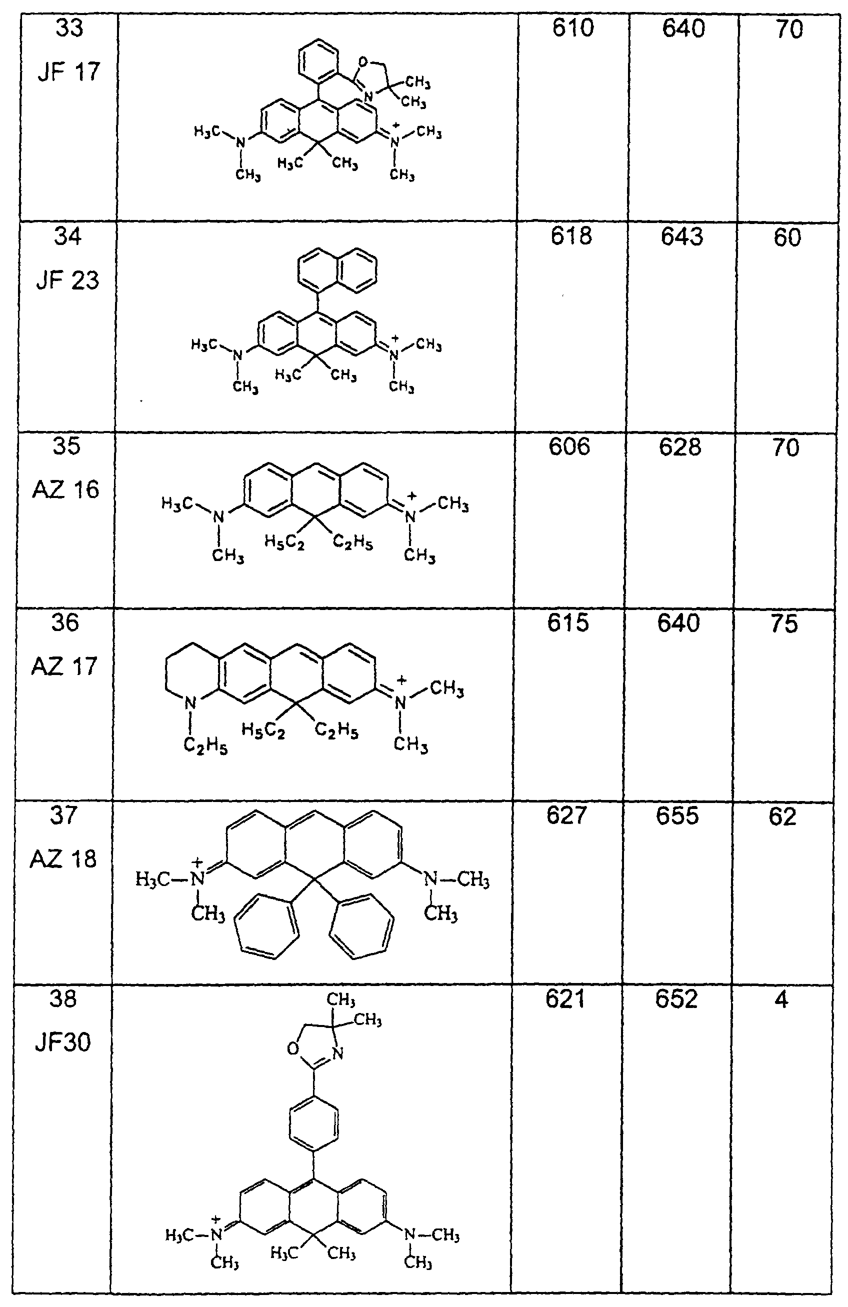

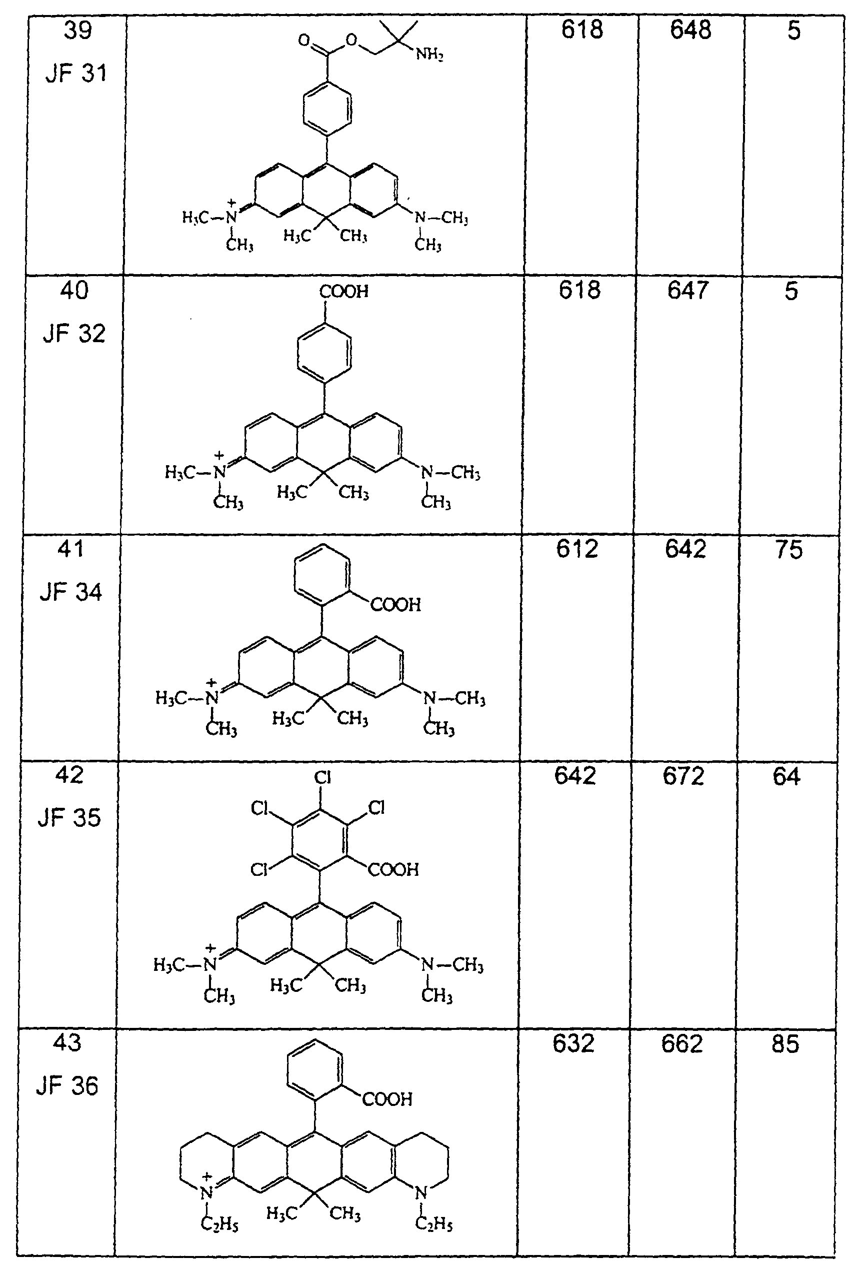

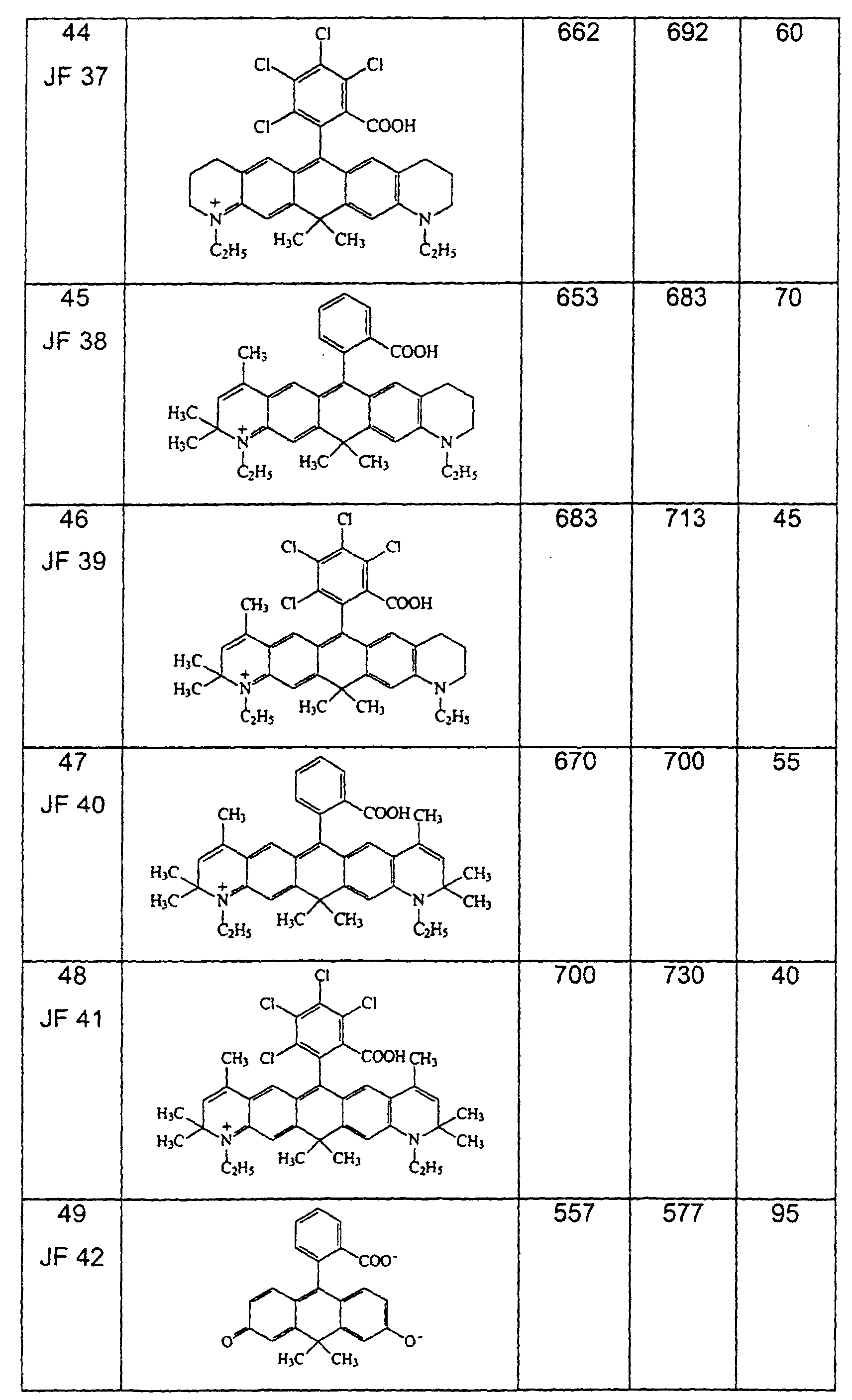

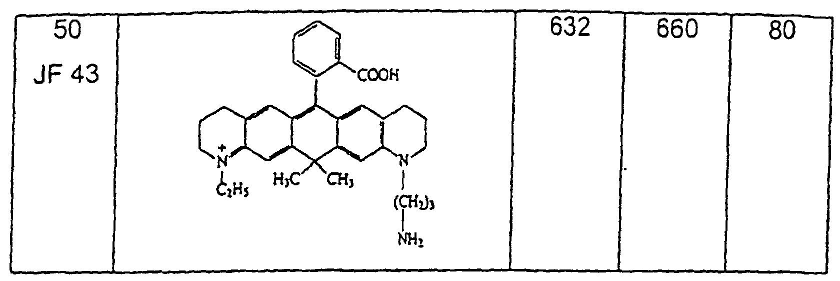

Eine weitere Aufgabe der vorliegenden Erfindung bestand in der Bereitstellung eines Herstellungsverfahrens für Carbopyronin-Verbindungen mit mindestens einem endständigen Ringsystem, das einfach, umweltverträglich und kostengünstig durchführbar ist, und das die Nachteile der bekannten Verfahren zur Herstellung von Carbopyroninen zumindest teilweise vermeidet.

Diese Aufgabe wurde erfindungsgemäß gelöst durch ein Verfahren zur Herstellung von Verbindungen der allgemeinen Formel (I) 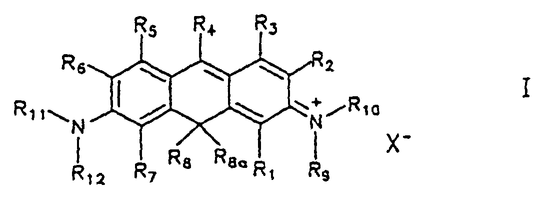 wobei R₁-R₁₂ und X die in Anspruch 1 angegebenen Bedeutungen besitzen, und R₆ mit R₁₁ oder/und R₇ mit R₁₂ oder/und R₁ mit R₁₀ oder/und R₂ mit R₉ verbrückt sind und ein Ringsystem bilden, das eine oder mehrere Mehrfachbindungen enthalten kann, dadurch gekennzeichnet, daß man eine Verbindung der allgemeinen Formel (II) 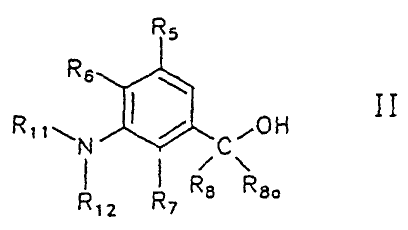 worin R₅, R₆, R₇, R₈, R₈ₐ, R₁₁ und R₁₂ wie oben definiert sind, und R₆ mit R₁₁ oder/und R₇ mit R₁₂ verbrückt sein können und ein Ringsystem bilden können, das eine oder mehrere Mehrfachbindungen enthalten kann, oder das Dehydratationsprodukt von II mit einer Verbindung der allgemeinen Formel III 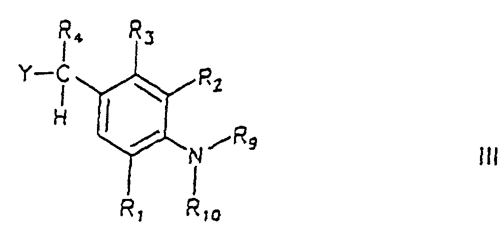 worin R₁-R₄, R₉ und R₁₀ wie oben definiert sind, und R₁ mit R₁₀ oder/und R₂ mit R₉ verbrückt sein können und ein Ringsystem bilden können, das eine oder mehrere Mehrfachbindungen enthalten kann und mindestens einer der Reste R₁₁ und R₁₂ in Verbindung II und R₉ und R₁₀ in Verbindung III mit benachbarten Substituenten ein Ringsystem bildet, und
Y ein Halogen, insbesondere Brom, eine Hydroxy- oder Thiogruppe bedeutet, in einem geeigneten Lösungsmittel, unter sauren Bedingungen und in Gegenwart eines Katalysators umsetzt und die durch Ringschluß zwischen den Verbindungen II bzw. deren Dehydratationsprodukt und III gebildete Verbindung durch Oxidation zu Struktur I umsetzt.

In dem Verfahren können alle geeigneten Lösungsmittel verwendet werden, die mit den Edukten, den Produkten und dem Katalysator, vorzugsweise Bortrichlorid, kompatibel sind. Vorzugsweise ist das Lösungsmittel ein unpolares Lösungsmittel, insbesondere Methylenchlorid, 1,2-Dichlorethan oder Chloroform.

Als Säuren können gebräuchliche Säuren eingesetzt werden. Vorzugsweise ist die Säure eine anorganische Säure wie Schwefelsäure, Phosphorsäure oder Polyphosphorsäure.

Als Oxidationsmittel können ebenfalls gebräuchliche Oxidationsmittel verwendet werden. Bevorzugt ist das Oxidationsmittel Tetrabutylammonium(meta)periodat.

Besonders vorteilhaft ist, daß das Verfahren ohne Isolierung von Zwischenprodukten durchgeführt werden kann. Dies führt zu einer Verringerung des Zeit-, Arbeits- und Materialaufwandes.

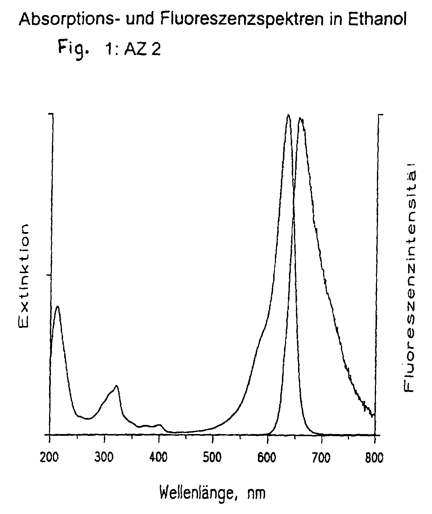

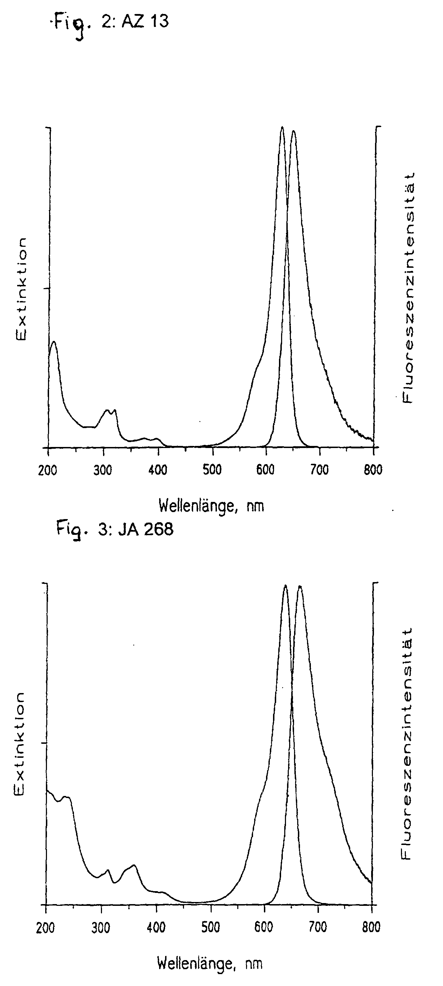

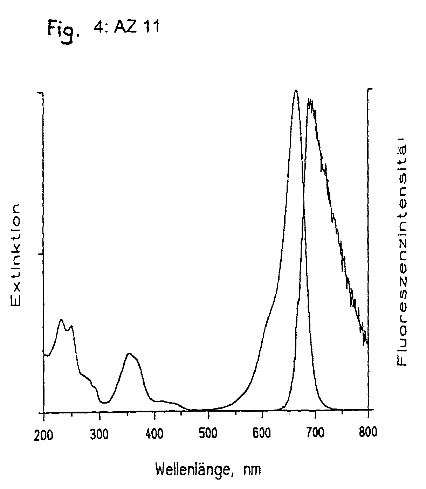

Die Erfindung wird durch nachstehende Beispiele näher erläutert. Die Abbildungen 1, 2, 3 und 4 zeigen die Absorptions- und Fluoreszenzspektren der erfindungsgemäßen Verbindungen AZ 2 (17), AZ 13 (20), JA 268 (16) und AZ11 (23).

### Beispiele

### A. Erfindungsgemäßes Herstellungsverfahren für Carbopyronin-Verbindungen

Bei dem erfindungsgemäßen Verfahren wird 4-N,N-Dimethylaminobenzylsulfanilsäure, die in dem Verfahren nach Aaron und Barker (J. Chem. Soc. (1963), 2655) verwendet wird, durch 4-Hydroxymethyl-N,N-dimethylanilin ersetzt und in Gegenwart von Bortrichloridlösung als Katalysator mit dem Isopropenylderivat zum Carbopyronin umgesetzt. Die Reaktionsmischung läßt sich ohne Isolierung des Zwischenproduktes mit konzentrierter Schwefelsäure zur Leukobase des Farbstoffs umsetzen. Das bei Aaron und Barker (a.a.o.) verwendete Oxidationsmittel Bleidioxid wird durch Tetrabutylammonium(meta)periodat ersetzt. Dazu wird die ethanolische Lösung aus Oxidationsmittel und Leukobase zum Sieden erhitzt, wobei dünnschichtchromatographisch zu erkennen ist, daß die Oxidation schon nach wenigen Minuten abgeschlossen ist.

Nach der Oxidation wird das Carbopyronin aus ethanolischer Lösung durch Zugabe von 10 %-iger Natriumperchloratlösung und langsames Zutropfen von Wasser als schwerlösliches Perchlorat ausgefällt.

Der neue Syntheseweg ist universell einsetzbar. Es können aus Anilin-, Indolin-, Tetrahydrochinolin- und 1,2-Dihydrochinolin-Derivaten durch eine Vilsmaier-Synthese mit anschließender Reduktion die entsprechenden Alkohole erhalten und diese mit einem Isopropenyl-Derivat zum Farbstoff umgesetzt werden. Im Gegensatz zur Synthese von Aaron und Barker verläuft die Synthese in einem Schritt, d.h. eine Isolierung von Zwischenprodukten ist nicht notwendig.

Die Synthesevorschriften für die Verbindungen JA 261, JA 262, AZ 4, AZ 14, JA 267, JA 268, JF 19, JF 22 und JF 17 sind im Folgenden dargestellt.

### B. Synthesebeispiele

### Verbindung JA 261

1 g (4 mmol) N-Methyl-N-(4-hydroxymethyl-phenyl)-4-aminobuttersäureethylester und 0,71 g (4,4 mmol) 3-(Isopropenyl)-N,N-dimethylanilin werden in 20 ml Methylenchlorid gelöst. Unter Rühren und Eiskühlung setzt man langsam 4 ml einer 1 molaren BCl₃-Lösung (in Methylenchlorid) zu. Die Lösung wird über Nacht bei Raumtemperatur verrührt. Anschließend tropft man die Reaktionsmischung in 20 g konzentrierte Schwefelsäure, welche im Eis/Methanol-Bad gekühlt wird. Man verrührt solange, bis eine homogene Lösung vorliegt. Das Methylenchlorid wird am Rotationsverdampfer abdestilliert. Die schwefelsaure Lösung wird über Nacht im Kühlschrank aufbewahrt. Anschließend wird die Lösung auf Eis gegossen und mit verdünnter Natronlauge neutralisiert. Die wässrige Lösung wird mit Chloroform extrahiert. Die vereinigten organischen Phasen werden über Natriumsulfat getrocknet, filtriert und zur Trockene einrotiert. Der Rückstand wird in 200 ml Ethanol aufgenommen, mit 10 Tropfen 60 %-iger Perchlorsäure und 0,17 g (0,39 mmol) Tetrabutylammonium(meta)periodat versetzt. Die Lösung wird 30 min zum Rückfluß erhitzt. Die abgekühlte Lösung wird in eine Lösung aus 20 g Natriumperchlorat in 1 l Wasser eingetropft. Man verrührt über Nacht. Der grün-glänzende Niederschlag wird abfiltriert und im Exsikkator über Phosphorpentoxid getrocknet.
Ausbeute: 0,56 g
¹H-NMR-Daten in CDCl₃:
δ 1,25 (T, 3H, -CH₃); 1,7 (S, 6H, -CH₃); 2,0 (Ql, 2H, -CH₂-); 2,5 (T, 2H, -CH₂-); 3,3 (S, 9H, N-CH₃); 3,7 (T, 2, -CH₂-); 4,15 (Q, 2H, N-CH₂-); 6,85 (DvD, 2H, ArH); 7,05 (D, 1H, ArH); 7,2 (D, 1H, ArH); 7,65 (D, 2H, Ar-H); 8,0 (S, 1H, -CH=)

### Verbindung JA 262

100 mg JA 261 werden in einer Mischung aus 20 ml Aceton, 40 ml Wasser und 2 ml 2 N Salzsäure gelöst. Die Lösung wird zum Rückfluß erhitzt (Innentemperatur: 64 °C). Nach 24 h wird die Lösung abgekühlt und mit 100 ml 10 %iger wässriger Natriumperchloratlösung versetzt. Der Niederschlag wird abfiltriert und getrocknet.
Ausbeute: 0,04 g

### Verbindung AZ 4

1,00 g (4,25 mmol) 4-(5-Hydroxymethylindolin-1-yl)-buttersäureethylester und 0,76 g (4,25 mmol) 3-(Isopropenyl)-N,N-dimethylanilin werden in 15 ml Methylenchlorid gelöst und unter Eiskühlung tropfenweise mit 4,25 ml (4,25 mmol) einer 1 molaren Lösung von Bortrichlorid in Hexan versetzt. Das Reaktionsgemisch wird 30 min bei Raumtemperatur verrührt. Anschließend tropft man das Reaktionsgemisch in 10 ml konzentrierte Schwefelsäure und läßt 1 h bei Raumtemperatur rühren. Die tief rot gefärbte Reaktionsmischung wird in 100 ml eiskaltes Ethanol getropft, mit 0,78 g (1,8 mmol) Tetrabuty-Iammonium(meta)periodat versetzt und 3 min zum Sieden erhitzt. Man läßt auf Raumtemperatur abkühlen und versetzt mit 50 ml 20 %-iger Natriumperchloratlösung. Anschließend werden zum vollständigen Ausfällen des Farbstoffes 300 ml Wasser zugetropft. Das kristalline Produkt wird abfiltriert und im Exsikkator unter Vakuum mit SICAPENT® getrocknet.
Ausbeute: 0,7 g
¹H-NMR-Daten in Aceton-d₆: 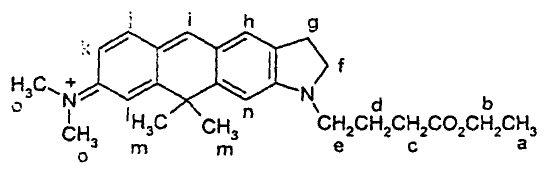 δ 0,9 (T, 3H, -CH₃ a); 1,7 (S, 6H, -CH₃ m); 2,47 (T, 2H, -CH₂- c); 3,22 (T, 2H, -CH₂- g); 3,34 (S, 6H, N-CH₃ o); 3,8 (T, 2, -CH₂- e); 4,09 (T, 2H, -CH₂- f); 4,42 (Q, 2H, -CH₂- b); 6,95 (DvD, 1H, ArH k); 7,22 (D, 1H, ArH l); 7,3 (S, 1H, ArH n); 7,7 (D, 1H, Ar-Hj); 8,08 (S, 1H, -CH= i)

### Verbindung AZ 14

4 g (8 mmol) AZ 4 werden in 30 ml Wasser und 20 ml Aceton gelöst und mit 1 ml 2 N Salzsäure versetzt. Das Reaktionsgemisch wird 18 h zum Rückfluß erhitzt. Man versetzt mit 50 ml Chloroform und trennt die organische Phase ab. Nach weiterer dreimaliger Extraktion mit Chloroform werden die vereinigten organischen Phasen mit Wasser gewaschen und über Natriumsulfat getrocknet. Die Farbstofflösung wird am Rotationsverdampfer bis zur Trokkene eingeengt und anschließend säulenchromatographisch gereinigt.
¹H-NMR-Daten in Aceton-d₆: 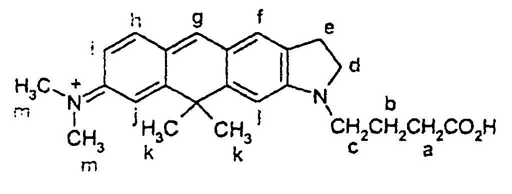 δ 1,72 (S, 6H, -CH₃ k); 2,0 (M, 2H, -CH₂- b); 2,49 (T, 3H, -CH₂- a); 3,25 (T, 2H, -CH₂- e); 3,34 (S, 6H, -CH₃ m); 3,81 (T, 2, -CH₂- c); 4,11 (T, 2H, -CH₂- d); 6,95 (DvD, 1H, ArH i); 7,22 (D, 1H, ArH j); 7,3 (S, 1H, ArH l); 7,42 (S, 1H, Ar-H f); 7,7 (D, 1H, ArH h); 8,1 (S, 1H, -CH= g)

### Verbindung JA 267

1,2 g (3,8 mmol) 4-(6-Hydroxymethyl-2,2,4-trimethyl-1,2-dihydrochinol-1-yl)-buttersäureethylester und 0,68 g (3,8 mmol) 3-(Isopropenyl)-N,N-dimethylanilin werden in 30 ml Methylenchlorid gelöst. Unter Rühren und Eiskühlung setzt man langsam 4 ml einer 1 molaren BCl₃-Lösung in Methylenchlorid zu. Die Lösung wird 20 min bei Raumtemperatur verrührt. Anschließend tropft man die Reaktionsmischung in 20 ml konz. Schwefelsäure. Man verrührt solange, bis eine homogene Lösung vorliegt. Das Methylenchlorid wird am Rotationsverdampfer abdestilliert und die schwefelsaure Lösung 1 h bei Raumtemperatur verrührt. Der Rückstand wird in 400 ml eisgekühltem Ethanol aufgenommen. Dazu gibt man 1,2 g (2,7 mmol) Tetrabutylammonium(meta)periodat. Die Lösung wird kurz zum Sieden erhitzt, abgekühlt und mit 200 ml einer 20 %igen Natriumperchlorat-Lösung versetzt. Anschließend tropft man 500 ml Wasser hinzu. Der Niederschlag wird abfiltriert und im Exsikkator getrocknet.

### Verbindung JA 268

1,8 g JA 267 werden in einer Mischung aus 50 ml Aceton, 50 ml Wasser und 5 ml 2 N Salzsäure 6 h zum Rückfluß erhitzt. Das Lösungsmittel wird abdestilliert und der Rückstand chromatographisch gereinigt.

### Verbindung JF 19

Zu einer Lösung von 50 mg (0,16 mmol) 2,10-Bis-(dimethylamino)-anthron in 10 ml trockenem Tetrahydrofuran werden innerhalb einer Argonschutzgasatmosphäre bei Raumtemperatur tropfenweise 0,27 ml (0,81 mmol) einer 3 M Methylmagnesiumbromidlösung in Diethylether gegeben. Nach beendeter Reaktion kühlt man im Eis-Wasser-Bad ab, löst die Reaktionsmischung in 50 ml Ethanol und säuert mit Trifluoressigsäure an. Diese Lösung wird in einer Mischung aus 50 ml Chloroform und 50 ml Wasser suspendiert. Die organische Phase wird abgetrennt, am Rotationsverdampfer zur Trockene eingeengt und in Ethanol gelöst. Anschließend wird in 100 ml wässrige 25 %-ige Natriumperchloratlösung getropft. Nach beendeter Zugabe werden noch 300 ml Wasser zugetropft. Der ausfallende Farbstoff wird filtriert und im Vakuum getrocknet.
Ausbeute: 0,04g

### Verbindung JF 22

Unter Argonschutz werden 11 mg (1,6 mmol) Lithiumpulver (0,5 % Natrium, Metallgesellschaft) in 2 ml trockenem Diethylether suspendiert. Hierzu tropft man unter Rühren eine Lösung von 0,17 g (0,8 mmol) 1-Brom-2,6-diethylbenzol in 4 ml Diethylether. Nach beendeter Zugabe läßt man 15 min bei Raumtemperatur rühren. Die Suspension wird über Glaswolle filtriert, um die verbleibenden Lithiumreste zu entfernen. Die so erhaltene Lösung wird bei Raumtemperatur zu einer Lösung von 50 mg (0,16 mmol) 2,10-Bis-(dimethylamino)-anthron in 10 ml trockenem Tetrahydrofuran getropft. Nach beendeter Reaktion kühlt man im Eis-Wasser-Bad ab, löst die Reaktionsmischung in 50 ml Ethanol und säuert mit Trifluoressigsäure an. Diese Lösung wird in einer Mischung aus 50 ml Chloroform und 50 ml Wasser suspendiert. Die organische Phase wird abgetrennt, am Rotationsverdampfer zur Trockene eingeengt und säulenchromatographisch über Kieselgel gereinigt. Nachdem die Farbstofffraktion am Rotationsverdampfer zur Trockene eingeengt wurde, wird in Ethanol gelöst und anschließend in 100 ml wässrige 25 %-ige Natriumperchloratlösung getropft. Dann werden noch 300 ml Wasser zugetropft. Der ausfallende Farbstoff wird filtriert und im Vakuum getrocknet.
Ausbeute: 0,02 g

### Verbindung JF 17

0,14 g (0,55 mmol) 2-(2-Bromphenyl)-4,4-dimethyl-2-oxazolin werden unter Schutzgas (Argon) in 7,5 ml Tetrahydrofuran gelöst und auf -78 °C abgekühlt. Hierzu gibt man tropfenweise 0,7 ml (1,1 mmol) einer 1,6 M Lösung von t-Butyllithium in Hexan, so daß die Temperatur unter - 75 °C bleibt. Nach beendeter Zugabe läßt man 15 min rühren. Zu dieser Lösung gibt man 34 mg (0.11 mmol) 2,10-Bis-(dimethylamino)-anthron in 2 ml trockenem Tetrahydrofuran. Die Temperatur sollte dabei - 70 °C nicht übersteigen. Anschließend wird auf -60 °C erwärmt und 3 h verrührt. Man entfernt das Kältebad und läßt auf Raumtemperatur erwärmen. Nach 24 h kühlt man im Eis-Wasser-Bad ab, löst die Reaktionsmischung in 50 ml Ethanol und säuert mit Trifluoressigsäure an. Diese Lösung wird in einer Mischung aus 50 ml Chloroform und 50 ml Wasser suspendiert. Die organische Phase wird abgetrennt, am Rotationsverdampfer zur Trockene eingeengt und säulenchromatographisch gereinigt. Die Farbstofffraktion wird am Rotationsverdampfer zur Trockene eingeengt, in Ethanol aufgenommen und anschließend in 100 ml wässrige 25 %-ige Natriumperchloratlösung getropft. Nach beendeter Zugabe werden noch 300 ml Wasser zugetropft. Der ausfallende Farbstoff wird filtriert und im Vakuum getrocknet.

### Verbindung AZ 18

### 1. Stufe:

### 3-(N,N-Dimethylamino)-triphenylcarbinol

2,8 g (0,12 mol) Magnesium und 10 ml Diethylether (absolut) werden mit 2,6 g (0,02 mol) Brombenzol versetzt. Um die Reaktion zu starten, wird leicht erwärmt. Der Reaktionsbeginn ist an der Trübung der Reaktionsmischung zu erkennen. Anschließend werden 16,2 g (0,1 mol) Brombenzol in 15 ml Ether gelöst und in die Reaktionsmischung getropft. Man erhitzt 1 h zum Rückfluß, wobei sich das Magnesium nahezu vollständig löst. Nach dem Abkühlen im Eisbad wird eine Lösung aus 10 g (0,055 mol) 3-Dimethylaminobenzoesäuremethylester in 15 ml absolutem Ether zugetropft. Nach der Zugabe wird die Reaktionsmischung 2 h zum Rückfluß erhitzt, abgekühlt und tropfenweise mit Wasser hydrolysiert. Man gibt 50 ml Wasser und 50 ml Ether zu und versetzt solange mit gesättigter Ammoniumchloridlösung, bis sich der weiße Niederschlag wieder gelöst hat. Die wässrige Phase wird mit Ether extrahiert. Die vereinigten organischen Phasen werden mit gesättigter Natriumhydrogencarbonatlösung und mit Wasser gewaschen. Anschließend wird über Natriumsulfat getrocknet und das Lösungsmittel abdestilliert. Das zurückbleibende hellgelbe Öl kann direkt für die nachfolgende Reaktion verwendet werden.

### 2. Stufe:

### AZ 18

0,6 g (3 mmol) N,N-Dimethyl-4-hydroxymethyl-anilin und 0,9 g (3 mmol) 3-(N,N-Dimethylamino)-triphenylcarbinol werden in 30 ml Methylenchlorid gelöst. Unter Rühren und Eiskühlung setzt man langsam 4 ml einer 1 molaren BCl₃-Lösung in Methylenchlorid zu. Die Lösung wird 2 h bei Raumtemperatur verrührt. Anschließend tropft man die Reaktionsmischung in 20 ml 70 %-ige Schwefelsäure. Das Methylenchlorid wird am Rotationsverdampfer abdestilliert und die schwefelsaure Lösung 20 h bei Raumtemperatur verrührt. Der Rückstand wird langsam in 100 ml eisgekühltem Ethanol gelöst. Dazu gibt man 1,2 g (2,7 mmol) Tetrabutylammonium(meta)periodat. Die Lösung wird kurz zum Sieden erhitzt, abgekühlt und mit 100 ml einer 20 %-igen Natriumperchlorat-Lösung versetzt. Anschließend tropft man 250 ml Wasser hinzu. Der Niederschlag wird abfiltriert und im Exsikkator getrocknet.

### Verbindung JF 30

1.85 ml (3.05 mmol) einer 15 %-igen t-Butyllithiumlösung (in n-Pentan) werden bei - 78 °C zu einer Lösung von 0.39 g (1.53 mmol) 2-(4-Brom-phenyl)-4,4-dimethyl-2-oxazolin in 20 ml trockenem Tetrahydrofuran gegeben, so daß die Temperatur unterhalb - 70 °C bleibt. Nach vollständiger Zugabe werden 150 mg (0.48 mmol) 3,6-Bis-(dimethylamino)-anthron in 30 ml trockenem Tetrahydrofuran zugegeben, so daß die Temperatur unterhalb von - 60 °C bleibt. Man läßt die Lösung auf Raumtemperatur erwärmen und 18 h bei Raumtemperatur rühren. Man kühlt im Eis-Wasser-Bad ab, löst die Reaktionsmischung in 50 ml Ethanol und säuert mit Trifluoressigsäure an. Diese Lösung wird in einer Mischung aus 50 ml Chloroform und 50 ml Wasser suspendiert. Die organische Phase wird abgetrennt, am Rotationsverdampfer zur Trockene eingeengt, und säulenchromatographisch über Kieselgel gereinigt. Der Farbstoff wird mit 15 %-igem ethanolischen Chloroform eluiert. Nachdem die Produktphase am Rotationsverdampfer zur Trockene eingeengt wurde, wird in Ethanol gelöst und anschließend in 100 ml wässriger 25 %-ige Natriumperchloratlösung getropft. Nach beendeter Zugabe werden noch 300 ml Wasser zugetropft. Der ausfallende Farbstoff wird filtriert und im Vakuumexsikkator über Phosphorpentoxid getrocknet.

Ausbeute: 50 % (krist. Substanz nach Chromatographie)

### Verbindung JF 31

80 mg (0.14 mmol) JF 30 werden in 10 ml einer 1 : 3 Mischung aus 2 M Salzsäure und Aceton 40 min unter Rückfluß erhitzt. Man läßt abkühlen und suspendiert in 50 ml einer 1:1 Mischung aus Chloroform und Wasser. Die Wasserphase wird mit gesättigter Natriumhydrogencarbonatlösung neutralisiert. Die organische Phase wird abgetrennt und die wässrige mehrmals mit 20 %-igem ethanolischem Chloroform extrahiert. Die vereinigten organischen Phasen werden einrotiert und säulenchromatographisch über Kieselgel gereinigt. Der Farbstoff wird mit 20 %-igem ethanolischen Chloroform eluiert. Nachdem die Produktphase am Rotationsverdampfer zur Trockene eingeengt wurde, wird in Ethanol gelöst und anschließend in 100 ml wässriger 25 %-ige Natriumperchloratlösung getropft. Nach beendeter Zugabe werden noch 300 ml Wasser zugetropft. Der ausfallende Farbstoff wird filtriert und im Vakuumexsikkator über Phosphorpentoxid getrocknet.

Ausbeute: 72 % (krist. Substanz nach Chromatographie)

### Verbindung JF 32

70 mg (0.12 mmol) JF 31 werden in einer 10 %-iger Natriumhydroxidlösung in 1:1 Ethanol und Wasser 1 h zum Rückfluß erhitzt. Man läßt abkühlen und suspendiert in einer 1:1 Mischung aus Chloroform und Wasser. Man stellt mit Trifluoressigsäure auf pH = 8 und trennt die organische Phase ab. Die wässrige Phase wird mehrmals mit 20 %-igem ethanolischem Chloroform extrahiert. Diese Extraktion wird sooft wiederholt, bis sich kaum noch Farbstoff in der wässrigen Phase befindet (Prüfung durch Ansäuern). Die vereinten organischen Phasen werden mit Trifluoressigsäure auf pH = 2 eingestellt, einrotiert und säulenchromatographisch über Kieselgel gereinigt. Der Farbstoff wird mit 10 %-igem ethanolischen Chloroform eluiert. Nachdem die Produktphase am Rotationsverdampfer zur Trockene eingeengt wurde, wird in Ethanol gelöst und anschließend in 100 ml wässrige 25 %-ige Natriumperchloratlösung getropft. Nach beendeter Zugabe werden noch 300 ml Wasser zugetropft. Der ausfallende Farbstoff wird filtriert und im Vakuumexsikkator über Phosphorpentoxid getrocknet.

Ausbeute: 57 % (krist. Substanz nach Chromatographie)

### Verbindung JF 42

70 mg (0.12 mmol) JF 17 werden in 30 ml einer Lösung von 3 g Natriumhydroxid in Ethanol / Wasser (1:1) 1 h zum Rückfluß erhitzt. Man läßt abkühlen und neutralisiert die Lösung mit halbkonzentrierter Salzsäure. Der Farbstoff wird anschließend durch Zutropfen von Wasser ausgefälllt. Man filtriert ab und trocknet das Produkt im Vakuumexsikkator über Phosphorpentoxid.

### Verbindung JF 36

25.3 g (0.1 mol) 6-(2-Carboxybenzoyl)-N-ethyl-1,2,3,4-tetrahydrochinolin und 20.1 (0.1 mol) N-Ethyl-7-isopropenyl-1,2,3,4-tetrahydrochinolin werden in 500 ml Dichlormethan gelöst und mit 60 g Phosphorpentoxid versetzt. Man erhitzt 2 h unter Rückfluß, läßt abkühlen und destilliert das Lösungsmittel im Vakuum ab. Der Rückstand wird mit konz. Schwefelsäure versetzt. Diese Lösung wird 30 min bei Raumtemperatur verrührt. Daraufhin wird die schwefelsaure Lösung in 1000 ml eisgekühltem Ethanol gegeben und tropfenweise mit 50 ml 60 %-ige Perchlorsäure und 5 l versetzt. Der ausgefallene Farbstoff wird abfiltriert und im Vakuumexsikkator über Phosphorpentoxid getrocknet.

### Verbindung JF 37

39.1 g (0.1 mol) 6-(2-Carboxy-3,4,5,6-tetrachlorbenzoyl)-N-ethyl-1,2,3,4-tetrahydrochinolin und 20.1 (0.1 mol) N-Ethyl-7-isopropenyl-1,2,3,4-tetrahydrochinolin werden in 500 ml Dichlormethan gelöst und mit 60 g Phosphorpentoxid versetzt. Man erhitzt 2 h unter Rückfluß, läßt abküheln und destilliert das Lösungsmittel im Vakuum ab. Der Rückstand wird mit konz. Schwefelsäure versetzt. Diese Lösung wird 30 min bei Raumtemperatur verrührt. Daraufhin wird die schwefelsaure Lösung in 1000 ml eisgekühltem Ethanol gegeben und tropfenweise mit 50 ml 60 %.ige Perchlorsäure und 5 I versetzt. Der ausgefallene Farbstoff wird abfiltriert und im Vakuumexsikkator über Phosphorpentoxid getrocknet.

### C. Beispiele zur Konjugatbildung

### JA 262-Aktivester

0,1 mmol JA 262 werden mit 0,2 mmol N-Hydroxysuccinimid und 0,2 mmol Dicyclohexylcarbodiimid in 20 ml Acetonitril gelöst. Man läßt 4 h bei Raumtemperatur rühren und rotiert das Produktgemisch ein. Die Reinigung erfolgt chromatographisch (HPLC, RP 18).

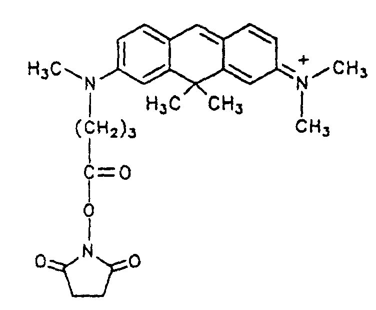

### JF 43-Maleinimid

100 mg JF 43 (0.16 mmol) werden in 10 ml getrocknetem DMSO gelöst und mit 100 mg (1 mmol) Maleinsäureanhydrid versetzt. Die Lösung wird bei Raumtemperatur für 24 h verrührt. Man tropft 50 ml 10 %-ige wässrige Natriumperchloratlösung hinzu und filtriert den ausfallenden Feststoff ab. Der Feststoff wird mit 25 mg Natriumacetat in 5 ml Essigsäureanhydrid suspendiert und für 30 min auf 80 °C erhitzt. Man kühlt ab und tropft 30 ml 10 %-ige wässrige Natriumperchloratlösung hinzu. Der Feststoff wird abfiltriert und getrocknet.

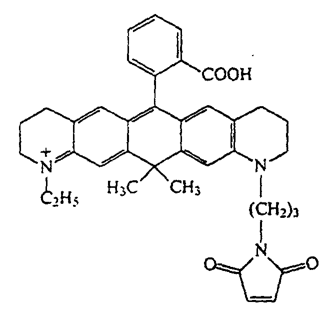

### JF43-Cystein-Konjugat

70 mg (0.1 mmol) JF 43-Maleinimid wird in 20 ml Ethanol gelöst und portionsweise mit 12 mg (0.1 mmol) Cystein versetzt. Man verrührt die Lösung 30 min bei Raumtemperatur. Daraufhin wird 50 ml 10 %-ige wässrige Natriumperchloratlösung hinzugetropft und der ausfallende Feststoff abfiltriert und getrocknet.

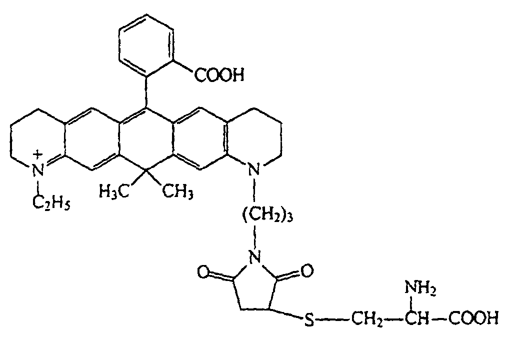

### JA 262-dUTP-Konjugat

10 µmol 5-(3-Aminoallyl)-dUTP werden in 0,5 ml 0,1 M Natriumborat-Puffer (pH 8) gelöst und mit einer Lösung aus 5 µmol JA 262-Aktivester in 1 ml aminfreiem Dimethylformamid versetzt. Die Lösung wird 15 h bei Raumtemperatur gerührt. Die Lösungsmittel werden im Vakuum abdestilliert und der Rückstand chromatographisch gereinigt (RP 18).

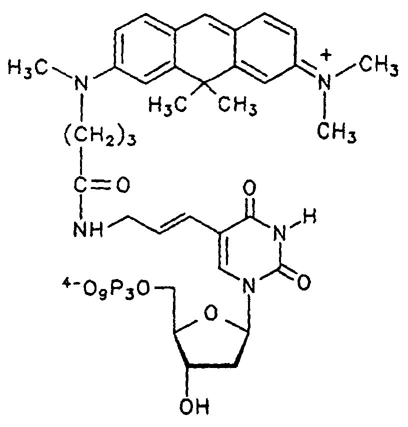

### JA 262-Digoxin-3-Carboxymethylether-Diaminodioxaoctan-Konjugat (Dig-CME-DADOO)

0,02 mmol JA 262-Aktivester werden mit 0,02 mmol Dig-CME-DADOO in Acetonitril 18 h bei Raumtemperatur verrührt. Das Lösungsmittel wird abdestilliert und der Rückstand chromatographisch gereinigt.

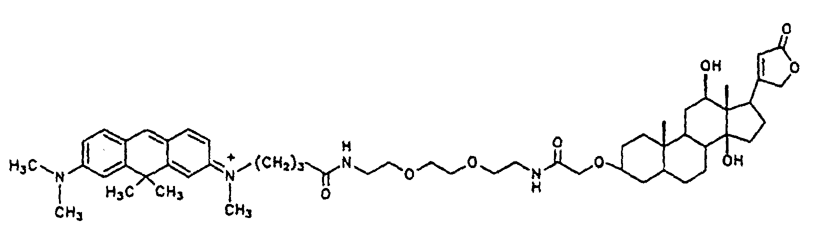

## Patentansprüche

1. Verwendung von Verbindungen der allgemeinen Formel l 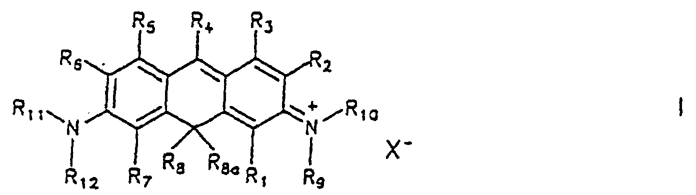 als Markierungsgruppen in einem Verfahren zum Nachweis eines Analyten, wobei
R₁, R₂, R₃, R₄, R₅, R₆, und R₇ jeweils unabhängig Wasserstoff, Halogen, eine Hydroxy-, Amino-, Sulfo- oder Carboxy- oder Aldehydgruppe oder eine gesättigte oder ungesättigte, geradkettige, verzweigte oder cyclische Kohlenwasserstoffgruppe mit bis zu 20 C-Atomen bedeuten, wobei die Kohlenwasserstoffgruppen Alkyl-, Alkenyl-, Alkinyl-, Cycloalkyl-, Aryl-, insbesondere Phenyl-, oder/und Heteroarylreste umfassen und gegebenenfalls Heteroatome wie Sauerstoff-, Schwefeloder Stickstoffatome oder/und mehrere Substituenten, vorzugsweise ausgewählt aus Halogenen, Hydroxy-, Amino-, Sulfo-, Phospho-, Carboxy-, Aldehyd-, C₁-C₄-Alkoxy- oder/und C₁-C₄-Alkoxycarbonylgruppen enthalten,
oder einer oder mehrere der Reste R₁-R₇ jeweils mit benachbarten Substituenten ein Ringsystem bilden, das eine oder mehrere Mehrfachbindungen enthalten kann,
R_{B} und R₈ₐ jeweils unabhängig eine gesättigte oder ungesättigte, geradkettige, verzweigte oder cyclische Kohlenwasserstoffgruppe mit bis zu 20 Kohlenstoffatomen, z.B. eine C₁-C₆-Alkylgruppe, insbesondere Methyl, Ethyl, Propyl oder/und Butyl, oder eine Aryl- oder Heteroarylgruppe, insbesondere Phenyl, bedeuten, die gegebenenfalls Heteroatome wie Sauerstoff-, Schwefel- oder Stickstoffatome oder/und einen oder mehrere Substituenten, vorzugsweise ausgewählt aus Halogenen, Hydroxy-, Amino-, Sulfo-, Phospho-, Carboxy-, Aldehyd-, C₁-C₄-Alkoxyoder/und C₁-C₄-Alkoxycarbonylgruppen, enthalten,
oder auch R₈ und R₈ₐ ein Ringsystem bilden können,
R₉, R₁₀, R₁₁ und R₁₂ jeweils unabhängig Wasserstoff oder eine gesättigte oder ungesättigte, geradkettige, verzweigte oder cyclische Kohlenwasserstoffgruppe mit bis zu 20 C-Atomen, z.B. Polyether, Phenyl, Phenylalkyl mit 1-3 C-Atomen in der Kette bedeuten, wobei die Kohlenwasserstoffgruppen gegebenenfalls Heteroatome wie Sauerstoff-, Schwefel- oder Stickstoffatome oder/und einen oder mehrere Substituenten, vorzugsweise ausgewählt aus Halogenen, Hydroxy-, Amino-, Sulfo-, Phospho-, Carboxy-, Carbonyl-, Alkoxy- oder/und Alkoxycarbonylgruppen enthalten können,
oder einer oder mehrere der Reste R₉-R₁₂ jeweils mit benachbarten Substituenten ein Ringsystem bilden, das eine oder mehrere Mehrfachbindungen enthalten kann,
wobei -N(R₁₁)(R₁₂) oder/und =N(R₉)(R₁₀) durch-OR⁹ oder/und = O ersetzt sein können,
und X gegebenenfalls zum Ladungsausgleich vorhandene Anionen bedeutet.

2. , Verwendung nach Anspruch 1,
**dadurch gekennzeichnet,**
**daß** die Verbindung I kovalent an einen für den nachzuweisenden Analyten spezifischen Rezeptor gekoppelt wird.

3. Verwendung nach Anspruch 1 oder 2,
**dadurch gekennzeichnet,**
**daß** das Nachweisverfahren aus Nukleinsäure-Hybridisierungsverfahren und immunchemischen Verfahren ausgewählt wird.

4. Verbindungen der allgemeinen Formel I 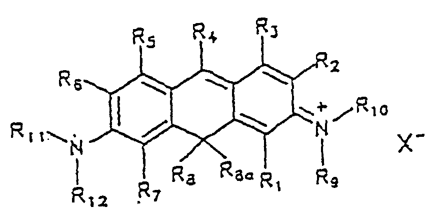 wobei R₁-R₁₂ und X die in Anspruch 1 angegebenen Bedeutungen besitzen, mit der Maßgabe, daß, wenn R₁-R₃ und R₅-R₇ Wasserstoff sind und R₈, R₈ₐ und R₉-R₁₂ Methyl sind,
R₄ nicht Wasserstoff, Hydroxyl, Methyl, Isopropyl, t-Butyl, Phenyl, o-Tolyl, p-Tolyl, 2,6-Dimethylphenyl, 2-t-Butylphenyl, 2-Isopropenylphenyl oder 4-Dimethylaminophenyl ist.

5. Verbindungen nach Anspruch 4,
**dadurch gekennzeichnet,**
**daß** R₆ mit R₁₁ oder/und R₇ mit R₁₂, R₁ mit R₁₀ oder/und R₂ mit R₉ verbrückt sind und ein Ringsystem, das vorzugsweise 5- oder 6-gliedrige Ringe enthält, bilden, welche eine oder mehrere Mehrfachbindungen enthalten können.

6. Verbindungen nach Anspruch 4 oder 5,
**dadurch gekennzeichnet,**
**daß** R₄ Wasserstoff, C₁-C₆-Alkyl oder einen ein aromatisches Ringsystem enthaltenden Rest bedeuten.

7. Verbindungen nach einem der Ansprüche 4 bis 6,
**dadurch gekennzeichnet,**
**daß** R₈ und R₈ₐ jeweils unabhängig Methyl, Ethyl oder/und Phenyl sind.

8. Verbindungen nach einem der Ansprüche 4 bis 6, die einer der allgemeinen Formeln IVa bis IVe entsprechen. 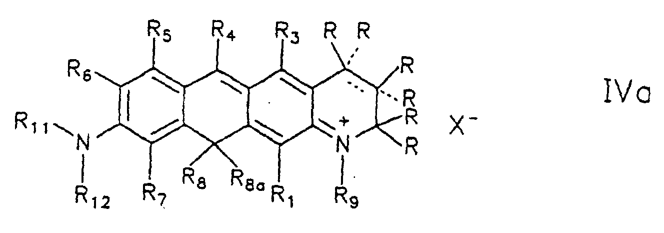 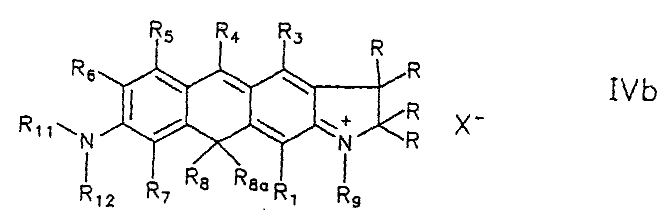 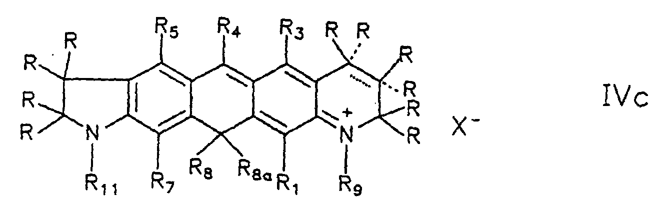 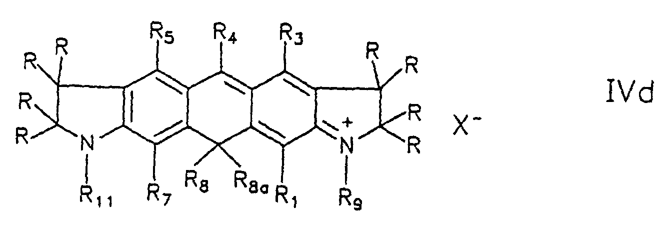 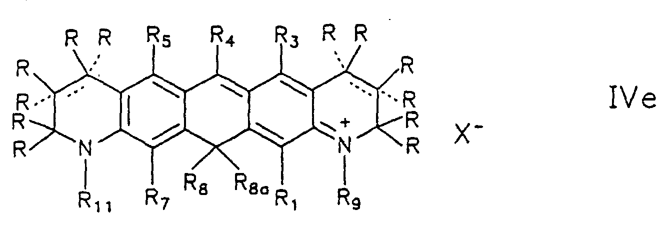 worin
die gestrichelten Linien gegebenenfalls Doppelbindungen bedeuten und bei Vorhandensein der Doppelbindungen die über eine gestrichelte Linie gebundenen Reste R fehlen,
R₁, R₃, R₄, R₅, R₆, R₇, R₈, R₈ₐ, R₉, R₁₁, R₁₂ und X wie in Anspruch 1 definiert sind,
und R bei jedem Auftreten gleich oder verschieden sein kann und wie R₁-R₇ in Anspruch 1 definiert ist.

9. Verbindungen nach einem der Ansprüche 4 bis 8,
**dadurch gekennzeichnet,**
**daß** sie eine zur kovalenten Kopplung fähige Gruppe aufweisen.

10. Verbindungen nach Anspruch 9,
**dadurch gekennzeichnet,**
**daß** die Kopplungsgruppe -COOH, -NH₂, -OH oder/und -SH ist.

11. Verbindungen nach Anspruch 9 oder 10,
**dadurch gekennzeichnet,**
**daß** sie über Kopplungsgruppen an einen Träger oder/und an ein Biomolekül gekoppelt sind.

12. Verbindungen nach Anspruch 10,
**dadurch gekennzeichnet,**
**daß** der Träger ausgewählt ist aus porösem Glas, lonenaustauschharzen, Dextranen, Cellulose, Cellulosederivaten oder/und hydrophilen Polymeren.

13. Verbindungen nach Anspruch 10,
**dadurch gekennzeichnet,**
**daß** das Biomolekül ausgewählt ist aus Peptiden, Polypeptiden, Nukleotiden, Nukleosiden, Nukleinsäuren, Nukleinsäureanaloga oder/und Haptenen.

14. Verfahren zur Herstellung von Verbindungen der allgemeinen Formel I 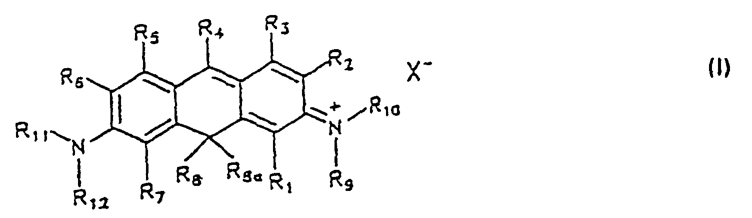 wobei R₁-R₁₂ und X die in Anspruch 1 angegebenen Bedeutungen besitzen, und R₆ mit R₁₁ oder/und R₇ mit R₁₂ oder/und R₁ mit R₁₀ oder/und R₂ mit R₉ verbrückt sind und ein Ringsystem bilden, das eine oder mehrere Mehrfachbindungen enthalten kann,
**dadurch gekennzeichnet,**
**dass** man eine Verbindung der allgemeinen Formel II 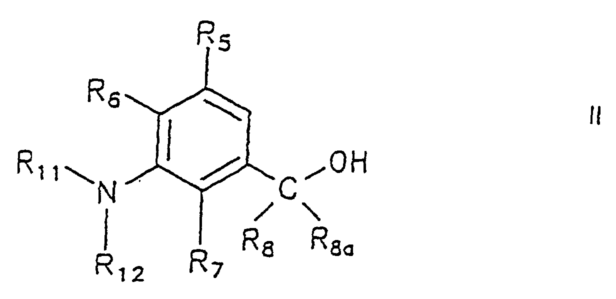 worin R₅, R₆, R₇, R₈, R₈ₐ, R₁₁ und R ₁₂ wie in Anspruch 1 definiert sind, und R₆ mit R₁₁ oder/und R₇ mit R₁₂ verbrückt sein können und ein Ringsystem bilden können, das eine oder mehrere Mehrfachbindungen enthalten kann, oder das Dehydratationsprodukt von II mit einer Verbindung der allgemeinen Formel III 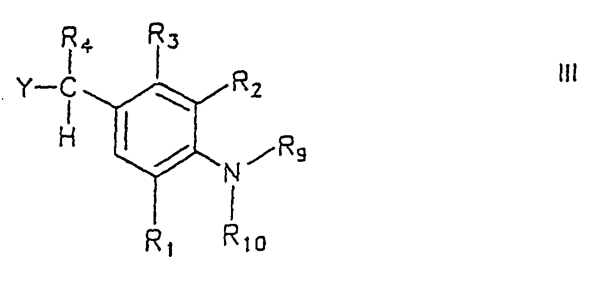 worin R₁-R₄, R₉ und R₁₀ wie in Anspruch 1 definiert sind, und R₁ mit R₁₀ oder/und R₂ mit R₉ verbrückt sein können und ein Ringsystem bilden können, das eine oder mehrere Mehrfachbindungen enthalten kann, und mindestens einer der Reste R₁₁ und R₁₂ in Verbindung II und R₉ und R₁₀ in Verbindung III mit benachbarten Substituenten ein Ringsystem bildet, und
Y ein Halogen, insbesondere Brom, eine Hydroxy- oder Thiogruppe bedeutet, worin R₁-R₄, R₉ und R₁₀ wie in Anspruch 1 definiert sind und Y ein Halogen,insbesondere Brom, eine Hydroxy- oder Thiogruppe bedeutet,in einem geeigneten Lösungsmittel, unter sauren Bedingungen und in Gegenwart eines Katalysators umsetzt und die durch Ringschluß zwischen der Verbindung II oder deren Dehydratationsprodukt und der Verbindung III gebildete Verbindung durch Oxidation zum Farbstoff I umsetzt.

15. Verfahren nach Anspruch 14,
**dadurch gekennzeichnet,**
**daß** das Lösungsmittel ein unpolares Lösungsmittel, insbesondere Methylenchlorid, 1,2-Dichlorethan oder Chloroform ist.

16. Verfahren nach einem der Ansprüche 14 bis 15,
**dadurch gekennzeichnet,**
**daß** der Katalysator Bortrichlorid ist.

17. Verfahren nach einem der Ansprüche 14 bis 16,
**dadurch gekennzeichnet,**
**daß** die Säure Schwefelsäure, Phosphorsäure oder Polyphosphorsäure ist.

18. Verfahren nach einem der Ansprüche 14 bis 17,
**dadurch gekennzeichnet,**
**daß** das Oxidationsmittel Tetrabutylammonium(meta)periodat ist.

19. Verfahren nach einem der Ansprüche 14 bis 18,
**dadurch gekennzeichnet,**
**daß** man die Verbindung (I) ohne Isolierung von Zwischenprodukten gewinnt.

## Claims

1. Use of compounds of the general formula I 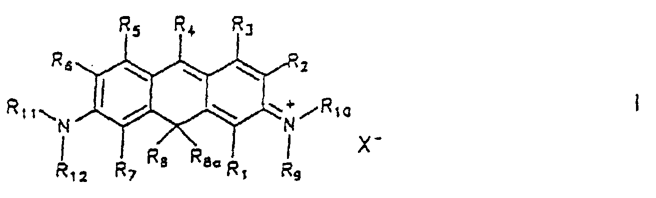 as labelling groups in a procedure for the detection of an analyte, where
R₁, R₂, R₃, R₄, R₅, R₆ and R₇ are in each case independently hydrogen, halogen, a hydroxyl, amino, sulpho or carboxyl or aldehyde group or a saturated or unsaturated, straight-chain, branched or cyclic hydrocarbon group having up to 20 C atoms, where the hydrocarbon groups include alkyl, alkenyl, alkynyl, cycloalkyl, aryl, in particular phenyl, or/and heteroaryl radicals and optionally heteroatoms such as oxygen, sulphur or nitrogen atoms or/and two or more substituents, preferably selected from halogens, hydroxyl, amino, sulpho, phospho, carboxyl, aldehyde, C₁-C₄-alkoxy or/and C₁-C₄-alkoxycarbonyl groups,
or one or more of the radicals R₁-R₇, in each case with adjacent substituents, form a ring system which can contain one or more multiple bonds,
R₈ and R₈ₐ in each case independently are a saturated or unsaturated, straight-chain, branched or cyclic hydrocarbon group having up to 20 carbon atoms, e.g. a C₁-C₆-alkyl group, in particular methyl, ethyl, propyl or/and butyl, or an aryl or heteroaryl group, in particular phenyl, which optionally contain heteroatoms such as oxygen, sulphur or nitrogen atoms or/and one or more substituents, preferably selected from halogens, hydroxyl, amino, sulpho, phospho, carboxyl, aldehyde, C₁-C₄-alkoxy or/and C₁-C₄-alkoxycarbonyl groups,
or alternatively R₈ and R₈ₐ can form a ring system,
R₉, R₁₀, R₁₁ and R₁₂ in each case independently are hydrogen or a saturated or unsaturated, straight-chain, branched or cyclic hydrocarbon group having up to 20 C atoms, e.g. polyether, phenyl, phenylalkyl having 1-3 C atoms in the chain, where the hydrocarbon groups can optionally contain heteroatoms such as oxygen, sulphur or nitrogen atoms or/and one or more substituents, preferably selected from halogens, hydroxyl, amino, sulpho, phospho, carboxyl, carbonyl, alkoxy or/and alkoxycarbonyl groups,
or one or more of the radicals R₉-R₁₂, in each case with adjacent substituents, form a ring system which can contain one or more multiple bonds,
where -N(R₁₁) (R₁₂) or/and =N(R₉) (R₁₀) can be replaced by -OR⁹ or/and =O,
and X is optionally anions present for charge equalization.

2. Use according to Claim 1,
**characterized in that**
the compound I is coupled covalently to a receptor specific for the analyte to be detected.

3. Use according to Claim 1 or 2,
**characterized in that**
the detection procedure is selected from nucleic acid hybridization procedures and immunochemical procedures.

4. Compound of the general formula I 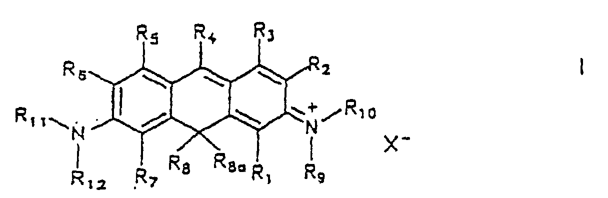 where R₁-R₁₂ and X have the meanings indicated in Claim 1, with the proviso that if R₁-R₃ and R₅-R₇ are hydrogen and R₈, R₈ₐ and R₉-R₁₂ are methyl,
R₄ is not hydrogen, hydroxyl, methyl, isopropyl, t-butyl, phenyl, o-tolyl, p-tolyl, 2,6-dimethylphenyl, 2-t-butylphenyl, 2-isopropenylphenyl or 4-dimethylaminophenyl.

5. Compound according to Claim 4,
**characterized in that**
R₆ is bridged with R₁₁ or/and R₇ with R₁₂, R₁ with R₁₀ or/and R₂ with R₉ to form a ring system which preferably contains 5- or 6-membered rings which can contain one or more multiple bonds.

6. Compound according to Claim 4 or 5,
**characterized in that**
R₄ is hydrogen, C₁-C₆-alkyl or a radical containing an aromatic ring system.

7. Compound according to one of Claims 4 to 6,
**characterized in that**
R₈ and R₈ₐ are in each case independently methyl, ethyl or/and phenyl.

8. Compound according to one of Claims 4 to 6, which corresponds to one of the general formulae IVa to IVe. 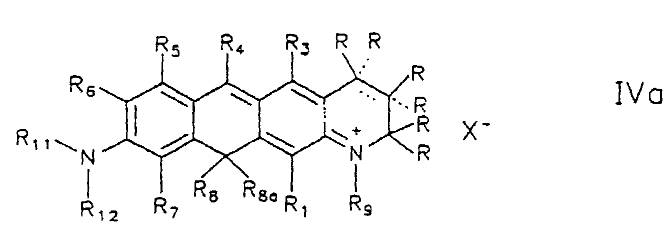 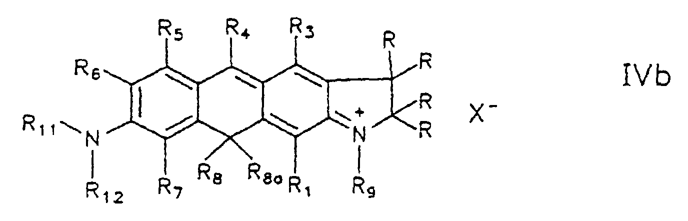 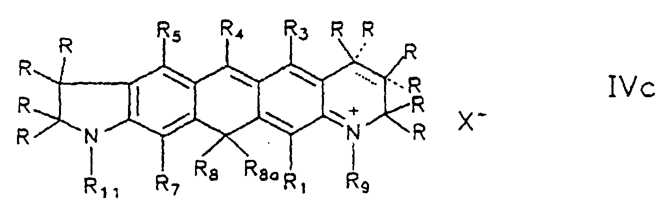 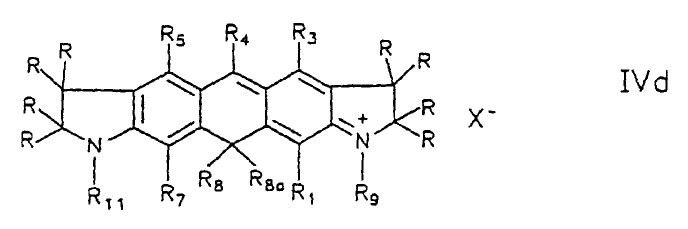 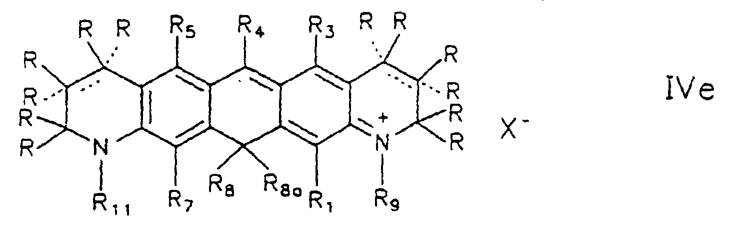 in which
the dashed lines are optionally double bonds, and in the presence of the double bonds the radicals R bonded via a dashed line are absent,
R₁, R₃, R₄, R₅, R₆, R₇, R₈, R₈ₐ, R₉, R₁₁, R₁₂ and X are as defined in Claim 1,
and R, on each occurrence, can be identical or different and is defined as R₁-R₇ in Claim 1.

9. Compound according to one of Claims 4 to 8,
**characterized in that**
it has a group capable of covalent coupling.

10. Compound according to Claim 9,
**characterized in that**
the coupling group is -COOH, -NH₂, -OH or/and -SH.

11. Compound according to Claim 9 or 10,
**characterized in that**
it is coupled to a carrier or/and to a biomolecule via coupling groups.

12. Compound according to Claim 10,
**characterized in that**
the carrier is selected from porous glass, ionexchange resins, dextrans, cellulose, cellulose derivatives or/and hydrophilic polymers.

13. Compound according to Claim 10,
**characterized in that**
the biomolecule is selected from peptides, polypeptides, nucleotides, nucleosides, nucleic acids, nucleic acid analogues or/and haptens.

14. Process for the preparation of compounds of the general formula I 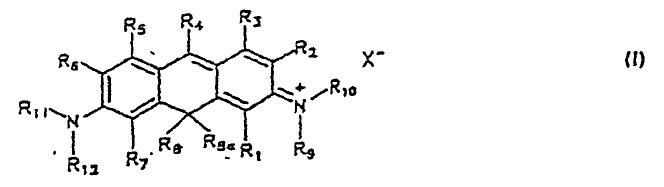 where R₁-R₁₂ and X have the meanings indicated in Claim 1, and R₆ is bridged with R₁₁ or/and R₇ with R₁₂ or/and R₁ with R₁₀ or/and R₂ with R₉ to form a ring system which can contain one or more multiple bonds,
**characterized in that**
a compound of the general formula II 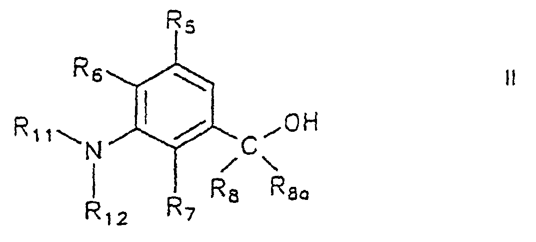 in which R₅, R₆, R₇, R₈, R₈ₐ, R₁₁, R₁₂ are as defined in Claim 1, and R₆ can be bridged with R₁₁ or/and R₇ with R₁₂ to form a ring system which can contain one or more multiple bonds, or the dehydration product of II is reacted with a compound of the general formula III 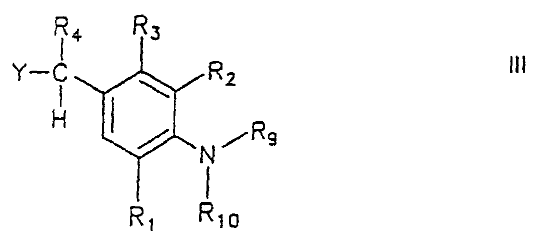 in which R₁-R₄, R₉ and R₁₀ are as defined in Claim 1, and R₁ can be bridged with R₁₀ or/and R₂ with R₉ to form a ring system which can contain one or more multiple bonds, and at least one of the radicals R₁₁ and R₁₂ in compound II and R₉ and R₁₀ in compound III combines with adjacent substituents to form a ring system, and Y is a halogen, in particular bromine, a hydroxyl or thio group,
in which R₁-R₄, R₉ and R₁₀ are as defined in Claim 1 and Y is a halogen, in particular bromine, a hydroxyl or thio group, in a suitable solvent, under acidic conditions and in the presence of a catalyst and the compound formed by ring closure between the compound II or its dehydration product and the compound III is reacted by oxidation to give the dye I.

15. Process according to Claim 14,
**characterized in that**
the solvent is a nonpolar solvent, in particular methylene chloride, 1,2-dichloroethane or chloroform.

16. Process according to one of Claims 14 to 15,
**characterized in that**
the catalyst is boron trichloride.

17. Process according to one of Claims 14 to 16,
**characterized in that**
the acid is sulphuric acid, phosphoric acid or polyphosphoric acid.

18. Process according to one of Claims 14 to 17,
**characterized in that**
the oxidant is tetrabutylammonium (meta)periodate.

19. Process according to one of Claims 14 to 18,
**characterized in that**
the compound (I) is obtained without isolation of intermediates.

## Revendications

1. Utilisation de composés de formule générale I : 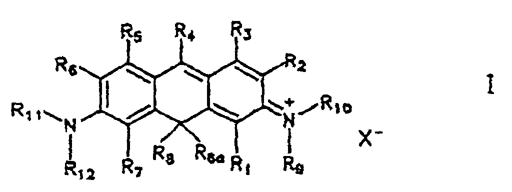 en tant que groupes de marquage dans un procédé d'identification d'un analyte, dans laquelle
R₁, R₂, R₃, R₄, R₅, R₆ et R₇, chacun indépendamment, représentent de l'hydrogène, un halogène, un groupe hydroxyle, amine, sulfanyle ou carboxyle ou aldéhyde ou un groupe hydrocarboné saturé ou insaturé, linéaire, ramifié ou cyclique avec jusqu'à 20 atomes de carbone, dans laquelle les groupes hydrocarbonés comprennent les résidus alkyle, alcényle, alcynyle, cycloalkyle, aryle, en particulier phényle, et/ou hétéroaryle et le cas échéant contiennent des hétéroatomes comme l'oxygène, le soufre ou l'azote et/ou plusieurs substituants, de préférence choisis parmi les groupes halogène, hydroxyle, amine, sulfanyle, phosphonyle, carboxyle, aldéhyde, alcoxyle en C₁-C₄ et/ou alcoxycarbonyle en C₁-C₄, ou un ou plusieurs des résidus R₁-R₇ chacun formant avec des substituants voisins un système cyclique qui peut présenter une ou plusieurs liaisons multiples,
R₈ et R₈ₐ, chacun indépendamment, représentent un groupe hydrocarboné saturé ou insaturé, linéaire, ramifié ou cyclique avec jusqu'à 20 atomes de carbone, par exemple un groupe alkyle en C₁-C₆, en particulier des groupes méthyle, éthyle, propyle et/ou butyle, ou un groupe aryle ou hétéroaryle, en particulier du phényle, qui le cas échéant contiennent des hétéroatomes comme de l'oxygène, du soufre ou de l'azote et/ou un ou plusieurs substituants, de préférence choisis parmi des groupes halogène, hydroxyle, amine, sulfanyle, phosphonyle, carboxyle, aldéhyde, alcoxyle en C₁-C₄ et/ou alcoxycarbonyle en C₁-C₄,
ou R₈ et R₈ₐ peuvent aussi former un système cyclique,
R₉, R₁₀, R₁₁ et R₁₂, chacun indépendamment, représentent l'hydrogène ou un groupe hydrocarboné saturé ou insaturé, linéaire, ramifié ou cyclique avec jusqu'à 20 atomes de carbone, par exemple un groupe polyéther, phényle, phénylalkyle avec une chaîne en C₁-C₃, dans lesquels les groupes hydrocarbonés peuvent contenir le cas échéant des hétéroatomes comme l'oxygène, le soufre ou l'azote et/ou un ou plusieurs substituants, de préférence choisis parmi des groupes halogène, hydroxyle, amine, sulfanyle, phosphonyle, carboxyle, carbonyle, alcoxyle et/ou alcoxycarbonyle,
ou un ou plusieurs des résidus R₉-R₁₂ chacun formant avec des substituants voisins un système cyclique qui peut présenter une ou plusieurs liaisons multiples,
dans laquelle -N(R₁₁) (R₁₂) et/ou =N(R₉) (R₁₀) peuvent être remplacé par -OR⁹ et/ou =O
et X représente le cas échéant des anions présents pour compenser la charge.

2. Utilisation selon la revendication 1, **caractérisée en ce que** le composé I est couplé par liaison covalente à un récepteur spécifique de l'analyte à identifier.

3. Utilisation selon la revendication 1 ou 2, **caractérisée en ce que** le procédé d'identification est choisi parmi un procédé d'hybridation et un procédé chimique immunitaire de l'acide nucléique.

4. Composés de formule générale I : 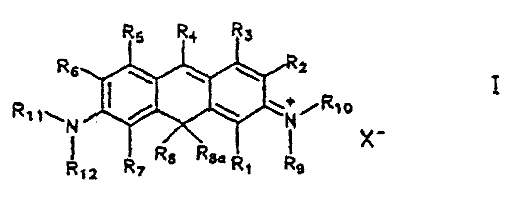 dans laquelle R₁-R₁₂ et X ont les significations données dans la revendication 1, sous réserve que, si R₁-R₃ et R₅-R₇ sont de l'hydrogène et R₈, R₈ₐ et R₉-R₁₂ sont un groupe méthyle,
R₄ ne soit pas de l'hydrogène, de l'hydroxyle, du métbyle, de l'isopropyle, du tert-butyle, du phényle, de l'o-tolyle, du p-tolyle, du 2,6-diméthylphényle, du 2-tert-butylphényle, du 2-isopropénylphényle ou du 4-diméthylaminophényle.

5. Composés selon la revendication 4, **caractérisés en ce que**, R₆ est ponté avec R₁₁ et/ou R₇ est ponté avec R₁₂, R₁ est ponté avec R₁₀ et/ou R₂ est ponté avec R₉ et forment un système cyclique qui contient de préférence des cycles de 5 ou 6 membres, lesquels peuvent contenir une ou plusieurs liaisons multiples.

6. Composés selon la revendication 4 ou 5, **caractérisés en ce que**, R₄ représente l'hydrogène, un groupe alkyle en C₁-C₆ ou un résidu contenant un système cyclique aromatique.

7. Composés selon l'une quelconque des revendications 4 à 6, **caractérisés en ce que** R₈ et R₈ₐ, chacun indépendamment, sont un groupe méthyle, éthyle, et/ou phényle.

8. Composés selon l'une quelconque des revendications 4 à 6, qui comprennent une des formules générales IVa à IVe 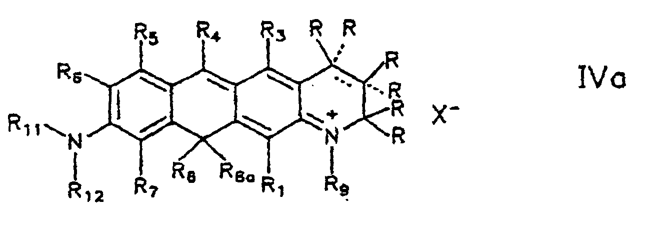 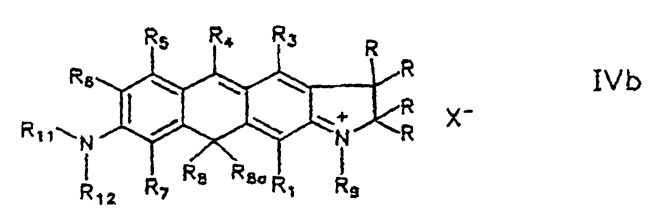 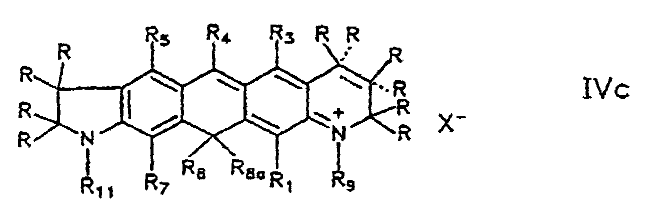 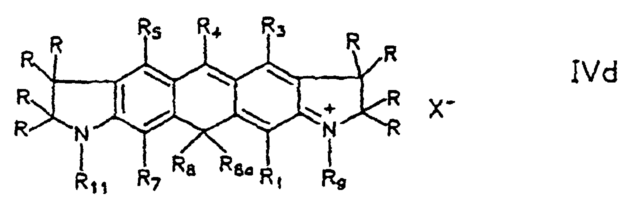 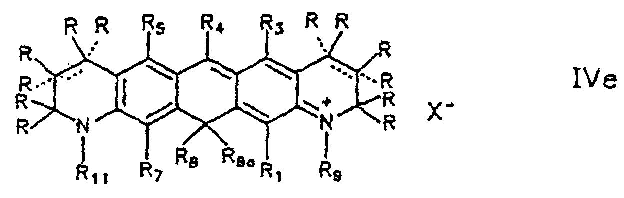 dans lesquelles
les lignes discontinues représentent le cas échéant des doubles liaisons et en cas de doubles liaisons les résidus R reliés par une ligne discontinue sont manquants, R₁, R₃, R₄, R₅, R₆, R₇, R₈, R₈ₐ, R₉, R₁₁, R₁₂ et X sont définis comme dans la revendication 1,
et R peut être identique ou différent dans chaque présentation et est défini comme R₁-R₇ dans la revendication 1.

9. Composés selon l'une quelconque des revendications 4 à 8, **caractérisés en ce qu'**ils représentent des groupes capables de couplage par liaison covalente.

10. Composés selon la revendication 9, **caractérisés en ce que** le groupe de couplage est -COOH, -NH₂, -OH et/ou -SH.

11. Composés selon la revendication 9 ou 10, **caractérisés en ce qu'**ils sont couplés par des groupes de couplage à un support et/ou à une biomolécule.

12. Composés selon la revendication 10, **caractérisés en ce que** le support est choisi parmi du verre poreux, des résines échangeuses d'ions, des dextranes, de la cellulose, des dérivés de la cellulose et/ou des polymères hydrophiles.

13. Composés selon la revendication 10, **caractérisés en ce que** la biomolécule est choisie parmi des peptides, des polypeptides, des nucléotides, des nucléosides, des acides nucléiques, des analogues de l'acide nucléique et/ou des haptènes.

14. Procédé de fabrication de composés de formule générale I 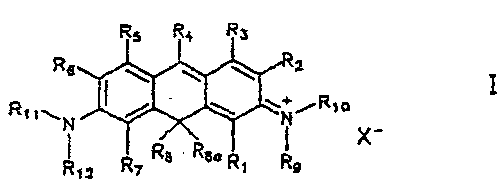 dans laquelle R₁-R₁₂ et X ont les significations données dans la revendication 1, et R₆ est ponté avec R₁₁ et/ou R₇ est ponté avec R₁₂ et/ou R₁ est ponté avec R₁₀ et/ou R₂ est ponté avec R₉ et forment un système cyclique qui peut contenir une ou plusieurs liaisons multiples, **caractérisé en ce que**,
un composé de formule générale II 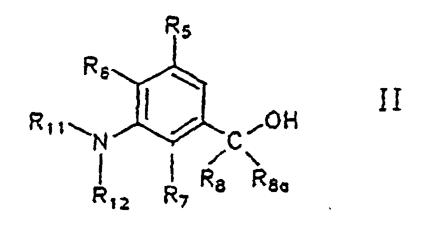 dans laquelle R₅, R₆, R₇, R₈, R₈ₐ, R₁₁, R₁₂ sont définis comme dans la revendication 1, et R₆ est ponté avec R₁₁ et/ou R₇ est ponté avec R₁₂ et peuvent former un système cyclique qui peut contenir une ou plusieurs liaisons multiples, ou le produit de la déshydratation de II avec un composé de formule générale III 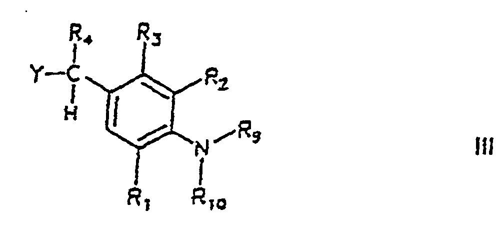 dans laquelle R₁-R₄, R₉ et R₁₀ sont définis comme dans la revendication 1, et R₁ est ponté avec R₁₀ et/ou R₂ est ponté avec R₉ et peuvent former un système cyclique qui peut contenir une ou plusieurs liaisons multiples, et au moins un des résidus R₁₁ et R₁₂ du composé II et R₉ et R₁₀ du composé III forme un système cyclique avec des substituants voisins, et
Y représente un halogène, en particulier du brome, un groupe hydroxyle ou thiol,
dans laquelle R₁-R₄, R₉ et R₁₀ sont définis comme dans la revendication 1, et Y représente un halogène, en particulier du brome, un groupe hydroxyle ou thiol, est mis à réagir dans un solvant approprié dans des conditions acides et en présence d'un catalyseur et le composé formé par cyclisation du composé II ou de son produit de déshydratation avec le composé III est transformé par oxydation en une matière colorante.

15. Procédé selon la revendication 14, **caractérisé en ce que** le solvant est un solvant non polaire, en particulier le chlorure de méthylène, le 1,2-dichloroéthane ou le chloroforme.

16. Procédé selon l'une des revendications 14 ou 15, **caractérisé en ce que** le catalyseur est le trichlorure de bore.

17. Procédé selon l'une des revendications 14 à 16, **caractérisé en ce que** l'acide est l'acide sulfurique, l'acide phosphorique ou l'acide polyphosphorique.

18. Procédé selon l'une des revendications 14 à 17, **caractérisé en ce que** l'agent d'oxydation est le méta-périodate de tétrabutylammonium.

19. Procédé selon l'une des revendications 14 à 18, **caractérisé en ce qu'**on obtient le composé (I) sans isolation de produits intermédiaires.
